(19) Europäisches Patentamt
European Patent Office
Office européen des brevets

(11) EP 2 788 346 B1

# (12) EUROPEAN PATENT SPECIFICATION

(45) Date of publication and mention of the grant of the patent:
**28.10.2015 Bulletin 2015/44**

(21) Application number: **12794731.5**

(22) Date of filing: **04.12.2012**

(51) Int Cl.:
***C07D 413/10*** (2006.01) ***C07D 413/12*** (2006.01)
***A61K 31/5377*** (2006.01) ***A61P 25/16*** (2006.01)
***A61P 25/28*** (2006.01)

(86) International application number:
**PCT/EP2012/074351**

(87) International publication number:
**WO 2013/083557 (13.06.2013 Gazette 2013/24)**

(54) **6-DIFLUOROMETHYL-5,6-DIHYDRO-2H-[1,4]OXAZIN-3-AMINE DERIVATIVES**

6-DIFLUORMETHYL-5,6-DIHYDRO-2H-[1,4]OXAZIN-3-AMIN-DERIVATE

DÉRIVÉS DE 6-DIFLUOROMÉTHYL-5,6-DIHYDRO-2H-[1,4]OXAZIN-3-AMINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **05.12.2011 EP 11191997**

(43) Date of publication of application:
**15.10.2014 Bulletin 2014/42**

(73) Proprietor: **Janssen Pharmaceutica, N.V.**
**2340 Beerse (BE)**

(72) Inventors:
- **TRABANCO-SUÁREZ, Andrés, Avelino**
  **E-45007 Toledo (ES)**
- **GIJSEN, Henricus, Jacobus, Maria**
  **B-2340 Beerse (BE)**
- **SURKYN, Michel**
  **B-2340 Beerse (BE)**
- **PROKOPCOVÁ, Hana**
  **B-2340 Beerse (BE)**

(74) Representative: **Quaghebeur, Luc**
**Janssen Pharmaceutica N.V.**
**Turnhoutseweg 30**
**B-2340 Beerse (BE)**

(56) References cited:
**WO-A1-2011/009943**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 788 346 B1

## Description

### FIELD OF THE INVENTION

The present invention relates to novel 6-difluoromethyl-5,6-dihydro-2*H*-[1,4]oxazin-3-amine derivatives as inhibitors of beta-secretase, also known as beta-site amyloid cleaving enzyme, BACE, BACE1, Asp2, or memapsin2. The invention is also directed to pharmaceutical compositions comprising such compounds, to processes for preparing such compounds and compositions, and to the use of such compounds and compositions for the prevention and treatment of disorders in which beta-secretase is involved, such as Alzheimer's disease (AD), mild cognitive impairment, senility, dementia, dementia with Lewy bodies, Down's syndrome, dementia associated with stroke, dementia associated with Parkinson's disease and dementia associated with beta-amyloid.

### BACKGROUND OF THE INVENTION

Alzheimer's Disease (AD) is a neurodegenerative disease associated with aging. AD patients suffer from cognition deficits and memory loss as well as behavioral problems such as anxiety. Over 90% of those afflicted with AD have a sporadic form of the disorder while less than 10% of the cases are familial or hereditary. In the United States, about 1 in 10 people at age 65 have AD while at age 85, 1 out of every two individuals are affected with AD. The average life expectancy from the initial diagnosis is 7-10 years, and AD patients require extensive care either in an assisted living facility which is very costly or by family members. With the increasing number of elderly in the population, AD is a growing medical concern. Currently available therapies for AD merely treat the symptoms of the disease and include acetylcholinesterase inhibitors to improve cognitive properties as well as anxiolytics and antipsychotics to control the behavioral problems associated with this ailment.

The hallmark pathological features in the brain of AD patients are neurofibillary tangles which are generated by hyperphosphorylation of tau protein and amyloid plaques which form by aggregation of beta-amyloid 1-42 (Abeta 1-42) peptide. Abeta 1-42 forms oligomers and then fibrils, and ultimately amyloid plaques. The oligomers and fibrils are believed to be especially neurotoxic and may cause most of the neurological damage associated with AD. Agents that prevent the formation of Abeta 1-42 have the potential to be disease-modifying agents for the treatment of AD. Abeta 1-42 is generated from the amyloid precursor protein (APP), comprised of 770 amino acids. The N-terminus of Abeta 1-42 is cleaved by beta-secretase (BACE), and then gamma-secretase cleaves the C-terminal end. In addition to Abeta 1-42, gamma-secretase also liberates Abeta 1-40 which is the predominant cleavage product as well as Abeta 1-38 and Abeta 1-43. These Abeta forms can also aggregate to form oligomers and fibrils. Thus, inhibitors of BACE would be expected to prevent the formation of Abeta 1-42 as well as Abeta 1-40, Abeta 1-38 and Abeta 1-43 and would be potential therapeutic agents in the treatment of AD.

WO-2011/009943 (Novartis) discloses unsubstituted and 2-substituted oxazine derivatives and their use as BACE inhibitors for the treatment of neurological disorders. WO-2011/020806 (Hoffmann-LaRoche) discloses 2,6-unsubstituted 3-amino-5-phenyl-5,6-dihydro-2H-[1,4]oxazine derivatives having BACE1 and/or BACE2 inhibitory properties.

### SUMMARY OF THE INVENTION

The present invention is directed to 5,6-dihydro-2*H*-[1,4]oxazin-3-amine derivatives of Formula (I)

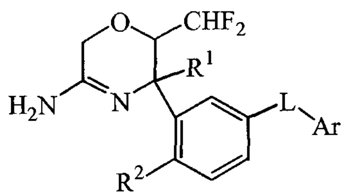

and the tautomers and the stereoisomeric forms thereof, wherein

$R^1$ is $C_{1-3}$alkyl;
$R^2$ is hydrogen or fluoro;
L is a bond or -NHCO-;
Ar is selected from the group consisting of pyridinyl, pyrimidinyl and pyrazinyl, each optionally susbstituted with halo or $C_{1-3}$alkoxy;

and the pharmaceutically acceptable addition salts thereof.

Illustrative of the invention is a pharmaceutical composition comprising a pharmaceutically acceptable carrier and any of the compounds described above. An illustration of the invention is a pharmaceutical composition made by mixing any of the compounds described above and a pharmaceutically acceptable carrier. Illustrating the invention is a process for making a pharmaceutical composition comprising mixing any of the compounds described above and a pharmaceutically acceptable carrier.

Exemplifying the invention are methods of treating a disorder mediated by the beta-secretase enzyme, comprising administering to a subject in need thereof a therapeutically effective amount of any of the compounds or pharmaceutical compositions described herein.

Further exemplifying the invention are methods of inhibiting the beta-secretase enzyme, comprising administering to a subject in need thereof a therapeutically effective amount of any of the compounds or pharmaceutical compositions described herein.

An example of the invention is a method of treating a disorder selected from the group consisting of Alzheimer's disease, mild cognitive impairment, senility, dementia, dementia with Lewy bodies, cerebral amyloid angiopathy, multi-infarct dementia, Down's syndrome, dementia associated with stroke, dementia associated with Parkinson's disease and dementia associated with beta-amyloid, preferably Alzheimer's disease, comprising administering to a subject in need thereof, a therapeutically effective amount of any of the compounds or pharmaceutical compositions described herein.

Another example of the invention is any of the compounds described above for use in treating: (a) Alzheimer's Disease, (b) mild cognitive impairment, (c) senility, (d) dementia, (e) dementia with Lewy bodies, (f) Down's syndrome, (g) dementia associated with stroke, (h) dementia associated with Parkinson's disease and (i) dementia associated with beta-amyloid, in a subject in need thereof.

## DETAILED DESCRIPTION OF THE INVENTION

The present invention is directed to compounds of Formula (I) as defined hereinbefore and pharmaceutically acceptable salts and solvates thereof. The compounds of Formula (I) are inhibitors of the beta-secretase enzyme (also known as beta-site cleaving enzyme, BACE, BACE1, Asp2 or memapsin 2), and are useful in the treatment of Alzheimer's disease, mild cognitive impairment, senility, dementia, dementia associated with stroke, dementia with Lewy bodies, Down's syndrome, dementia associated with Parkinson's disease and dementia associated with beta-amyloid, preferably Alzheimer's disease, mild cognitive impairment or dementia, more preferably Alzheimer's disease.

In an embodiment of the invention, $R^1$ is methyl or ethyl.

In an embodiment of the invention Ar is selected from 5-methoxy-pyridinyl, 5-pyrimidinyl and 5-fluoropyrazinyl.

In another embodiment of the invention, $R^2$ is hydrogen or fluoro.

In another embodiment, the quaternary carbon atom substituted with $R^1$ has the *R*-configuration.

## DEFINITIONS

"Halo" shall denote fluoro, chloro and bromo; "$C_{1-3}$alkyloxy" shall denote an ether radical wherein $C_{1-3}$alkyl is a straight or branched saturated alkyl group having 1, 2 or 3 carbon atoms, e.g. methyl, ethyl, 1-propyl and 2-propyl.

The term "subject" as used herein, refers to an animal, preferably a mammal, most preferably a human, who is or has been the object of treatment, observation or experiment.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of the disease or disorder being treated.

As used herein, the term "composition" is intended to encompass a product comprising the specified ingredients in the specified amounts, as well as any product which results, directly or indirectly, from combinations of the specified ingredients in the specified amounts.

Hereinbefore and hereinafter, the term "compound of formula (I)" is meant to include the addition salts, the solvates and the stereoisomers thereof.

The terms "stereoisomers" or "stereochemically isomeric forms" hereinbefore or hereinafter are used interchangeably.

The invention includes all stereoisomers of the compound of Formula (I) either as a pure stereoisomer or as a mixture of two or more stereoisomers.

Enantiomers are stereoisomers that are non-superimposable mirror images of each other. A 1:1 mixture of a pair of enantiomers is a racemate or racemic mixture. Diastereomers (or diastereoisomers) are stereoisomers that are not enantiomers, i.e. they are not related as mirror images. Therefore, the invention includes enantiomers, diastereomers,

racemates.

The absolute configuration is specified according to the Cahn-Ingold-Prelog system. The configuration at an asymmetric atom is specified by either R or S. Resolved compounds whose absolute configuration is not known can be designated by (+) or (-) depending on the direction in which they rotate plane polarized light.

When a specific stereoisomer is identified, this means that said stereoisomer is substantially free, i.e. associated with less than 50%, preferably less than 20%, more preferably less than 10%, even more preferably less than 5%, in particular less than 2% and most preferably less than 1%, of the other isomers. Thus, when a compound of formula (I) is for instance specified as (R), this means that the compound is substantially free of the (S) isomer.

The compounds of Formula (I) co-exist in a dynamic equilibrium with the tautomers of Formula (I-a).

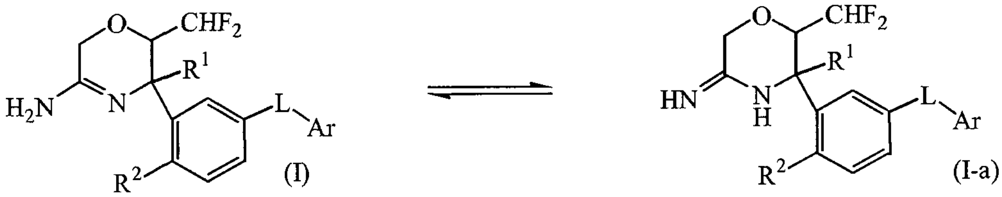

Furthermore, some of the crystalline forms for the compounds of the present invention may exist as polymorphs and as such are intended to be included in the present invention. In addition, some of the compounds of the present invention may form solvates with water (i.e., hydrates) or common organic solvents, and such solvates are also intended to be encompassed within the scope of this invention.

For use in medicine, the salts of the compounds of this invention refer to nontoxic "pharmaceutically acceptable salts". Other salts may, however, be useful in the preparation of compounds according to this invention or of their pharmaceutically acceptable salts. Suitable pharmaceutically acceptable salts of the compounds include acid addition salts which may, for example, be formed by mixing a solution of the compound with a solution of a pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid. Furthermore, where the compounds of the invention carry an acidic moiety, suitable pharmaceutically acceptable salts thereof may include alkali metal salts, e.g., sodium or potassium salts; alkaline earth metal salts, e.g., calcium or magnesium salts; and salts formed with suitable organic ligands, e.g., quaternary ammonium salts.

Representative acids which may be used in the preparation of pharmaceutically acceptable salts include, but are not limited to, the following: acetic acid, 2,2-dichloroacetic acid, acylated amino acids, adipic acid, alginic acid, ascorbic acid, L-aspartic acid, benzenesulfonic acid, benzoic acid, 4- acetamidobenzoic acid, (+)-camphoric acid, camphorsulfonic acid, capric acid, caproic acid, caprylic acid, cinnamic acid, citric acid, cyclamic acid, ethane-1,2-disulfonic acid, ethanesulfonic acid, 2-hydroxyethanesulfonic acid, formic acid, fumaric acid, galactaric acid, gentisic acid, glucoheptonic acid, D-gluconic acid, D-glucoronic acid, L-glutamic acid, beta-oxoglutaric acid, glycolic acid, hippuric acid, hydrobromic acid, hydrochloric acid, (+)-L-lactic acid, (±)-DL-lactic acid, lactobionic acid, maleic acid, (-)-L-malic acid, malonic acid, (±)-DL-mandelic acid, methanesulfonic acid, naphthalene-2-sulfonic acid, naphthalene-1,5- disulfonic acid, 1-hydroxy-2-naphthoic acid, nicotinic acid, nitric acid, oleic acid, orotic acid, oxalic acid, palmitic acid, pamoic acid, phosphoric acid, L- pyroglutamic acid, salicylic acid, 4-amino-salicylic acid, sebacic acid, stearic acid, succinic acid, sulfuric acid, tannic acid, (+)-L-tartaric acid, thiocyanic acid, p-toluenesulfonic acid, trifluoromethylsulfonic acid, and undecylenic acid. Representative bases which may be used in the preparation of pharmaceutically acceptable salts include, but are not limited to, the following: ammonia, L-arginine, benethamine, benzathine, calcium hydroxide, choline, dimethylethanolamine, diethanolamine, diethylamine, 2-(diethylamino)-ethanol, ethanolamine, ethylenediamine, *N*-methyl-glucamine, hydrabamine, 1*H*-imidazole, L-lysine, magnesium hydroxide, 4-(2-hydroxyethyl)-morpholine, piperazine, potassium hydroxide, 1-(2-hydroxyethyl)-pyrrolidine, secondary amine, sodium hydroxide, triethanolamine, tromethamine and zinc hydroxide.

The names of the compounds of the present invention were generated according to the nomenclature rules agreed upon by the Chemical Abstracts Service (CAS) using Advanced Chemical Development, Inc., software (ACD/Name product version 10.01; Build 15494, 1 Dec 2006) or according to the nomenclature rules agreed upon by the International Union of Pure and Applied Chemistry (IUPAC) using Advanced Chemical Development, Inc., software (ACD/Name product version 10.01.0.14105, October 2006). In case of tautomeric forms, the name of the depicted tautomeric form of the structure was generated. The other non-depicted tautomeric form is also included within the scope of the present invention.

Preparation of the compounds

Experimental procedure 1

The final compounds according to Formula (I) can be prepared by catalytic hydrogenation of an intermediate compound of Formula (II-a) according to reaction scheme (1). Said conversion may be conducted by treatment of the intermediate compound of Formula (II-a) with hydrogen in the presence of a suitable catalyst such as, for example, palladium on carbon, a suitable catalyst poison, such as, for example, thiophene, in a suitable reaction-inert solvent, such as, for example, ethyl acetate or methanol. The mixture is stirred under hydrogen atmosphere, at a suitable temperature, typically room temperature, at a suitable pressure, such as, for example, atmospheric pressure, for example for 16 hours. In reaction scheme (1), all variables are defined as in Formula (I).

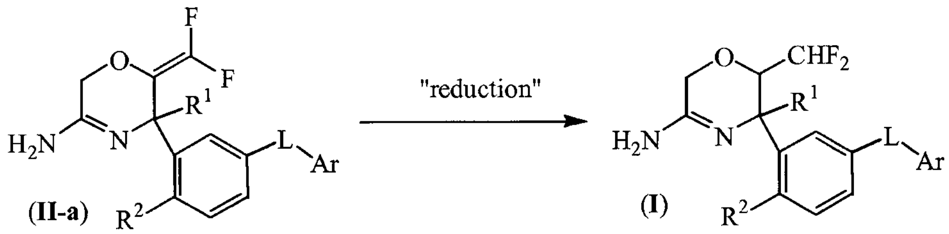


Reaction Scheme 1

Experimental procedure 2

The intermediate compounds of Formula (II-b) can generally be prepared by reacting an intermediate compound of Formula (III) with a compound of Formula (IV) according to reaction scheme (2), a reaction that is performed in a suitable reaction-inert solvent, such as, for example, dichloromethane or methanol, in the presence of a suitable base, such as, for example, triethylamine, in the presence of a condensation agent such as for example *O*-(7azabenzotriazol-1-yl)-*N,N,N',N'*-tetramethyluronium hexafluorophosphate [HATU, CAS 148893-10-1] or 4-(4,6-dimethoxy-1,3,5-triazin-2-yl)-4-methylmorpholinium chloride [DMTMM, CAS 3945-69-5] under thermal conditions such as, for example, heating the reaction mixture at 25 °C, for the required time to achieve completion of the reaction, for example 1-16 hours. In reaction scheme (2), all variables are defined as in Formula (I).

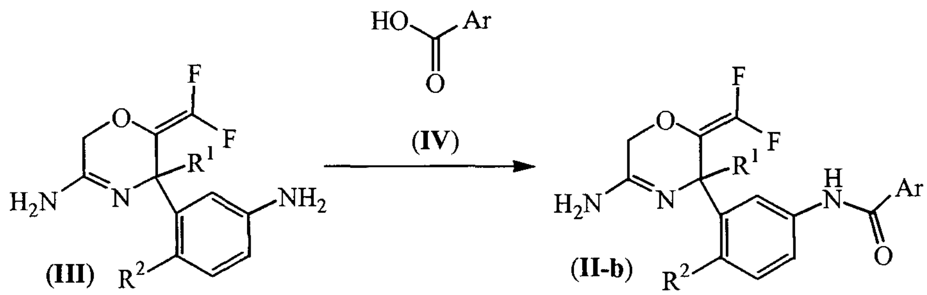


Reaction Scheme 2

Experimental procedure 3

Intermediate compounds of Formula (II-c) can generally be prepared by the reaction of intermediate compounds of Formula (VI) with an appropriate aryl-boronate or aryl boronic acid in a Suzuki type reaction. Thus intermediate compounds of Formula (VI) can react with an aryl-boronate or aryl boronic acid in a suitable reaction-inert solvent, such as, for example, 1,4-dioxane, ethanol or mixtures of inert solvents such as, for example, 1,2-dimethoxyethane/water/ethanol, in the presence of a suitable base, such as, for example, aqueous $K_3PO_4$, $Na_2CO_3$ or $Cs_2CO_3$, a Pd-complex catalyst such as, for example, [1,1'-bis(diphenylphosphino)ferrocene]dichloropalladium(II) [CAS 72287-26-4] or trans-bisdicyclohexylamine)palladium diacetate [DAPCy, CAS 628339-96-8] or tetrakis(triphenylphosphine) palladium (0) [CAS14221-01-3] under thermal conditions such as, for example, heating the reaction mixture at 80 °C, for example for a period of time between 2-20 hours or for example , heating the reaction mixture at 130 °C, for example for 10 minutes under microwave irradiation. In reaction scheme (3), all variables are defined as in Formula (I) and W is halo. $R^3$ and $R^4$ may be hydrogen or alkyl, or may be taken together to form for example a bivalent radical of formula $-CH_2CH_2-$,

$-CH_2CH_2CH_2-$, or $-C(CH_3)_2C(CH_3)_2-$.

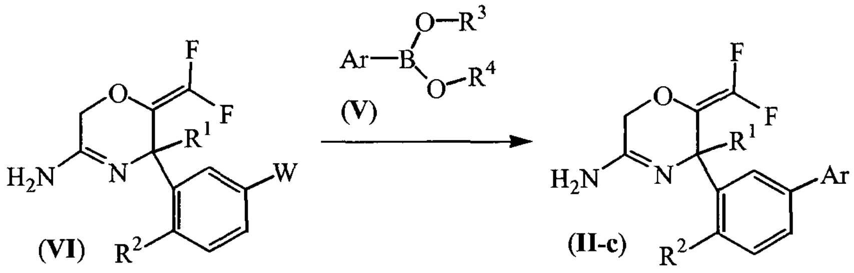

Reaction Scheme 3

Experimental procedure 4

The intermediate compounds of Formula (III) can generally be prepared following the reaction steps shown in the reaction scheme (4) below.

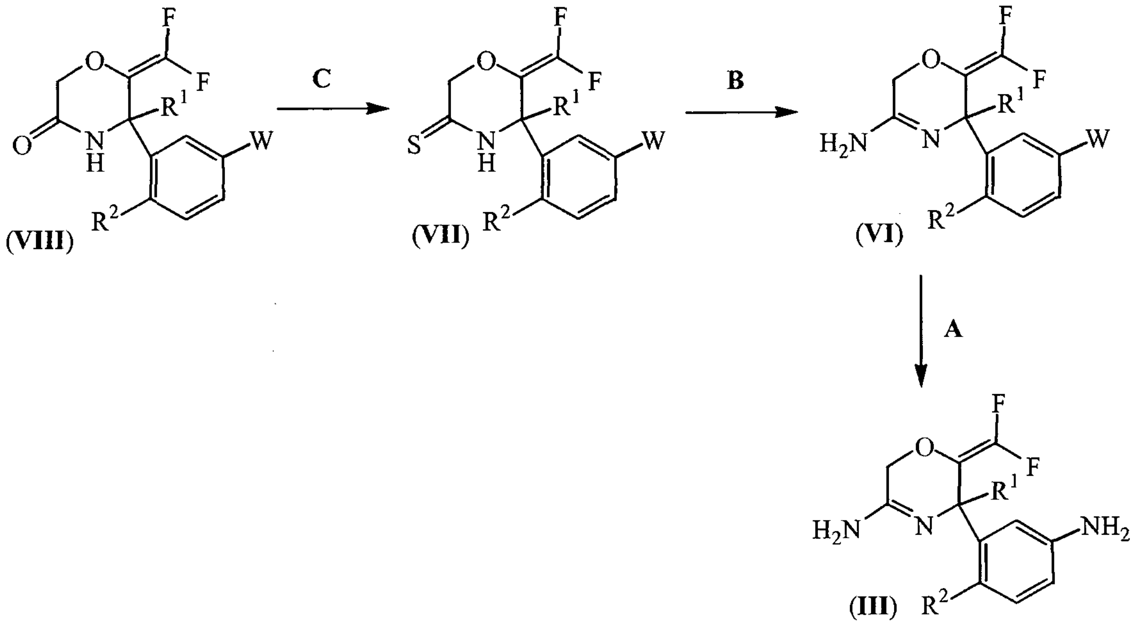


Reaction Scheme 4

**A**: Bromo-to-amine conversion

**B**: thioamide-to-amidine conversion

**C**: amide-to-thioamide conversion (thionation)

Intermediate compounds of Formula (III) in the above reaction scheme (4) can be prepared from the corresponding intermediate compounds of Formula (VI) following art-known copper catalyzed type coupling procedure (reaction step A). Said coupling may be conducted by treatment of said intermediate compounds of Formula (VI) with sodium azide in a suitable reaction-inert solvent, such as, for example, DMSO, in the presence of a mixture of suitable bases, such as, for example, dimethylethylenediamine and $Na_2CO_3$, and a copper catalyst such as, CuI, under thermal conditions such as, for example, heating the reaction mixture at 110 °C, until completion of the reaction, for example 1 hour.

Intermediate compounds of Formula (VI) in the above reaction scheme (4) can be prepared from the corresponding intermediate compounds of Formula (VII) following art-known thioamide-to-amidine conversion procedures (reaction step B). Said conversion may conveniently be conducted by treatment of intermediate compounds of Formula (VII) with an ammonia source such as, for example, ammonium chloride or aqueous ammonia, in a suitable reaction-inert solvent such as, for example, water or methanol and the like, under thermal conditions such as, for example, heating

the reaction mixture at 60 °C, for example for 6 hours.

Intermediate compounds of Formula (VII) in the above reaction scheme (4) can be prepared from the corresponding intermediate compounds of Formula (VIII) following art-known thionation procedures (reaction step C). Said conversion may conveniently be conducted by treatment of intermediate compounds of Formula (VIII) with a thionation agent such as, for example, phosphorous pentasulfide or 2,4-bis-(4-methoxyphenyl)-1,3-dithia-2,4-diphosphetane 2,4-disulfide [Lawesson's reagent, CAS 19172-47-5], in a reaction inert solvent such as, for example, tetrahydrofuran or 1,4-dioxane and the like, under thermal conditions such as, for example, heating the reaction mixture at 50 °C, for example for 50 minutes.

Experimental procedure 5

The intermediate compounds of Formula (VIII) and (IX) can generally be prepared from intermediate compounds of Formula (X) following art-known reductive dehalogenation procedures (reaction step D). Said conversion may be conducted by treatment of the intermediate of Formula (X) with a suitable zinc reagent, such as, for example, zinc dust or zinc copper couple in a suitable solvent, such as acetic acid, at a suitable temperature, typically from room temperature to 80 °C, for the required time to achieve completion of the reaction, for example 1-16 hours. This conversion affords a mixture of the intermediate compounds of Formula (VIII) and (IX) in different ratio depending on the reaction conditions and the reactants.

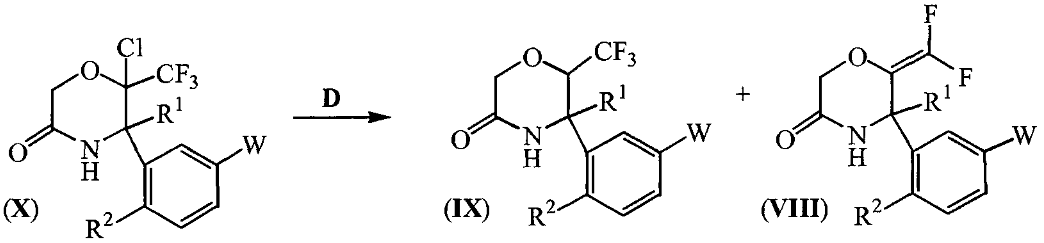


(X) (IX) (VIII)

Reaction Scheme 5

Experimental procedure 6

The intermediate compounds of Formula (X) can generally be prepared following the reaction steps shown in the reaction scheme (6) below.

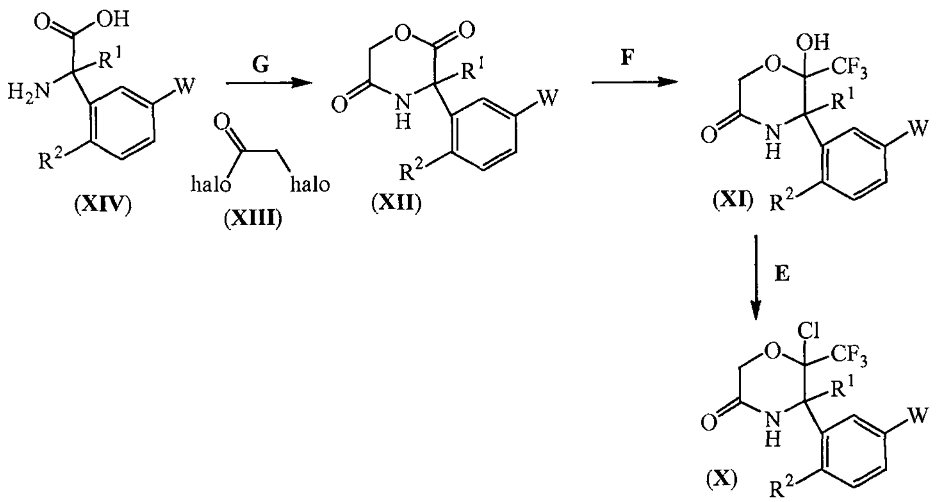


(XIV) (XIII) (XII) (XI) (X)

Reaction Scheme 6

E: chlorination

F: trifluoromethylation

G: cyclization

Intermediate compounds of Formula (X) in the above reaction scheme (6) can be prepared from intermediate compounds of Formula (XI) following art-known chlorination procedures (reaction step E). Said conversion may be conducted by treatment of the intermediate compound of Formula (XI) with a suitable chlorinating agent such as, for example, thionyl chloride, in the presence of a base such as, for example, pyridine in a reaction-inert solvent, such as, for example, dichloromethane. The reaction mixture is stirred at suitable temperature, for example 0 °C for the required time to achieve completion of the reaction, for example 30-60 minutes.

Intermediate compounds of Formula (XI) of the above reaction scheme (6) can be prepared from intermediate compounds of Formula (XII) following art-known trifluoromethylation procedures (reaction step F). Said conversion may be conducted by treatment of the intermediate compound of Formula (XII) in the presence of tetrabutyl ammonium fluoride (TBAF) or tetrabutyl ammonium triphenyldifluorosilicate (TBAT), with a trifluoromethylating agent such as, for example, (trifluoromethyl)trimethyl silane, in a suitable reaction-inert solvent, such as, for example, tetrahydrofuran. The reaction mixture is stirred at suitable temperature, for example room temperature for the required time to achieve completion of the reaction, for example two hours.

Intermediate compounds of Formula (XII) in the above reaction scheme (6) can be prepared from intermediate compounds of Formula (XIV) following art-known two-step cyclization procedures (reaction step G). Said conversion may be conducted by first, treatment of the intermediate compounds of Formula (XIV) with an intermediate compound of Formula (XIII), such as, for example, chloroacetylchloride in the presence of a base such as, for example, NaOH or DIPEA, in a suitable reaction-inert solvent, such as, for example, dichloromethane, or mixtures of inert solvents such as, for example, water and 1,4-dioxane or water and THF. The pH of the reaction mixture may be adjusted to a suitable pH value, for example, 10-11, by addition of a suitable base such as, for example, NaOH. The reaction mixture is stirred at a suitable temperature, for example, 0 °C to 25 °C for the required time to achieve completion of the reaction, for example 1-4 hours. The obtained crude residue can subsequently be cyclised to provide the intermediate (XII) by the addition of a suitable base such as, for example, $K_2CO_3$, $Cs_2CO_3$, *N,N*-diisopropylethylamine or $NaHCO_3$, in a suitable reaction-inert solvent, such as for example, acetonitrile or DMF. The reaction mixture is stirred under thermal conditions such as, for example, heating the reaction mixture at 25 °C to 80°C for 2-24 hours or for example, heating the reaction mixture at 140 °C for 15-30 minutes under microwave irradiation. This conversion can also be performed in the absence of a base in a suitable reaction-inert solvent, such as for example, acetonitrile or DMF, at a suitable temperature, typically 40 °C to 110 °C, for a period of, for example, 24-48 hours.

## PHARMACOLOGY

The compounds of the present invention and the pharmaceutically acceptable compositions thereof inhibit BACE and therefore may be useful in the treatment or prevention of Alzheimer's Disease (AD), mild cognitive impairment (MCI), senility, dementia, dementia with Lewy bodies, cerebral amyloid angiopathy, multi-infarct dementia, Down's syndrome, dementia associated with Parkinson's disease and dementia associated with beta-amyloid.

The invention relates to a compound according to the general Formula (I), a stereoisomeric form thereof or a pharmaceutically acceptable acid or base addition salt thereof, for use as a medicament.

The invention also relates to a compound according to the general Formula (I), a stereoisomeric form thereof or a the pharmaceutically acceptable acid or base addition salt thereof, for use in the treatment or prevention of diseases or conditions selected from the group consisting of AD, MCI, senility, dementia, dementia with Lewy bodies, cerebral amyloid angiopathy, multi-infarct dementia, Down's syndrome, dementia associated with Parkinson's disease and dementia associated with beta-amyloid.

The invention also relates to the use of a compound according to the general Formula (I), a stereoisomeric form thereof or a pharmaceutically acceptable acid or base addition salt thereof, for the manufacture of a medicament for the treatment or prevention of any one of the disease conditions mentioned hereinbefore.

In view of the utility of the compound of Formula (I), there is provided a method of treating subjects such as warm-blooded animals, including humans, suffering from or a method of preventing subjects such as warm-blooded animals, including humans, to suffer from any one of the diseases mentioned hereinbefore.

Said methods comprise the administration, i.e. the systemic or topical administration, preferably oral administration, of an effective amount of a compound of Formula (I), a stereoisomeric form thereof, a pharmaceutically acceptable addition salt or solvate thereof, to a subject such as a warm-blooded animal, including a human.

A method of treatment may also include administering the active ingredient on a regimen of between one and four intakes per day. In these methods of treatment the compounds according to the invention are preferably formulated prior to administration. As described herein below, suitable pharmaceutical formulations are prepared by known procedures using well known and readily available ingredients.

The compounds of the present invention, that can be suitable to treat or prevent Alzheimer's disease or the symptoms thereof, may be administered alone or in combination with one or more additional therapeutic agents. Combination therapy includes administration of a single pharmaceutical dosage formulation which contains a compound of

Formula (I) and one or more additional therapeutic agents, as well as administration of the compound of Formula (I) and each additional therapeutic agents in its own separate pharmaceutical dosage formulation. For example, a compound of Formula (I) and a therapeutic agent may be administered to the patient together in a single oral dosage composition such as a tablet or capsule, or each agent may be administered in separate oral dosage formulations.

**PHARMACEUTICAL COMPOSITIONS**

The present invention also provides compositions for preventing or treating diseases in which inhibition of beta-secretase is beneficial, such as Alzheimer's disease (AD), mild cognitive impairment, senility, dementia, dementia with Lewy bodies, Down's syndrome, dementia associated with stroke, dementia associated with Parkinson's disease and dementia associated with beta-amyloid. Said compositions comprising a therapeutically effective amount of a compound according to formula (I) and a pharmaceutically acceptable carrier or diluent.

While it is possible for the active ingredient to be administered alone, it is preferable to present it as a pharmaceutical composition. Accordingly, the present invention further provides a pharmaceutical composition comprising a compound according to the present invention, together with a pharmaceutically acceptable carrier or diluent. The carrier or diluent must be "acceptable" in the sense of being compatible with the other ingredients of the composition and not deleterious to the recipients thereof.

The pharmaceutical compositions of this invention may be prepared by any methods well known in the art of pharmacy. A therapeutically effective amount of the particular compound, in base form or addition salt form, as the active ingredient is combined in intimate admixture with a pharmaceutically acceptable carrier, which may take a wide variety of forms depending on the form of preparation desired for administration. These pharmaceutical compositions are desirably in unitary dosage form suitable, preferably, for systemic administration such as oral, percutaneous or parenteral administration; or topical administration such as via inhalation, a nose spray, eye drops or via a cream, gel, shampoo or the like. For example, in preparing the compositions in oral dosage form, any of the usual pharmaceutical media may be employed, such as, for example, water, glycols, oils, alcohols and the like in the case of oral liquid preparations such as suspensions, syrups, elixirs and solutions: or solid carriers such as starches, sugars, kaolin, lubricants, binders, disintegrating agents and the like in the case of powders, pills, capsules and tablets. Because of their ease in administration, tablets and capsules represent the most advantageous oral dosage unit form, in which case solid pharmaceutical carriers are obviously employed. For parenteral compositions, the carrier will usually comprise sterile water, at least in large part, though other ingredients, for example, to aid solubility, may be included. Injectable solutions, for example, may be prepared in which the carrier comprises saline solution, glucose solution or a mixture of saline and glucose solution. Injectable suspensions may also be prepared in which case appropriate liquid carriers, suspending agents and the like may be employed. In the compositions suitable for percutaneous administration, the carrier optionally comprises a penetration enhancing agent and/or a suitable wettable agent, optionally combined with suitable additives of any nature in minor proportions, which additives do not cause any significant deleterious effects on the skin. Said additives may facilitate the administration to the skin and/or may be helpful for preparing the desired compositions. These compositions may be administered in various ways, e.g., as a transdermal patch, as a spot-on or as an ointment.

It is especially advantageous to formulate the aforementioned pharmaceutical compositions in dosage unit form for ease of administration and uniformity of dosage. Dosage unit form as used in the specification and claims herein refers to physically discrete units suitable as unitary dosages, each unit containing a predetermined quantity of active ingredient calculated to produce the desired therapeutic effect in association with the required pharmaceutical carrier. Examples of such dosage unit forms are tablets (including scored or coated tablets), capsules, pills, powder packets, wafers, injectable solutions or suspensions, teaspoonfuls, tablespoonfuls and the like, and segregated multiples thereof.

The exact dosage and frequency of administration depends on the particular compound of formula (I) used, the particular condition being treated, the severity of the condition being treated, the age, weight, sex, extent of disorder and general physical condition of the particular patient as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

Depending on the mode of administration, the pharmaceutical composition will comprise from 0.05 to 99 % by weight, preferably from 0.1 to 70 % by weight, more preferably from 0.1 to 50 % by weight of the active ingredient, and, from 1 to 99.95 % by weight, preferably from 30 to 99.9 % by weight, more preferably from 50 to 99.9 % by weight of a pharmaceutically acceptable carrier, all percentages being based on the total weight of the composition.

The present compounds can be used for systemic administration such as oral, percutaneous or parenteral administration; or topical administration such as via inhalation, a nose spray, eye drops or via a cream, gel, shampoo or the like. The compounds are preferably orally administered. The exact dosage and frequency of administration depends on the particular compound according to formula (I) used, the particular condition being treated, the severity of the condition being treated, the age, weight, sex, extent of disorder and general physical condition of the particular patient

as well as other medication the individual may be taking, as is well known to those skilled in the art. Furthermore, it is evident that said effective daily amount may be lowered or increased depending on the response of the treated subject and/or depending on the evaluation of the physician prescribing the compounds of the instant invention.

The amount of a compound of Formula (I) that can be combined with a carrier material to produce a single dosage form will vary depending upon the disease treated, the mammalian species, and the particular mode of administration. However, as a general guide, suitable unit doses for the compounds of the present invention can, for example, preferably contain between 0.1 mg to about 1000 mg of the active compound. A preferred unit dose is between 1 mg to about 500 mg. A more preferred unit dose is between 1 mg to about 300mg. Even more preferred unit dose is between 1 mg to about 100 mg. Such unit doses can be administered more than once a day, for example, 2, 3, 4, 5 or 6 times a day, but preferably 1 or 2 times per day, so that the total dosage for a 70 kg adult is in the range of 0.001 to about 15 mg per kg weight of subject per administration. A preferred dosage is 0.01 to about 1.5 mg per kg weight of subject per administration, and such therapy can extend for a number of weeks or months, and in some cases, years. It will be understood, however, that the specific dose level for any particular patient will depend on a variety of factors including the activity of the specific compound employed; the age, body weight, general health, sex and diet of the individual being treated; the time and route of administration; the rate of excretion; other drugs that have previously been administered; and the severity of the particular disease undergoing therapy, as is well understood by those of skill in the area.

A typical dosage can be one 1 mg to about 100 mg tablet or 1 mg to about 300 mg taken once a day, or, multiple times per day, or one time-release capsule or tablet taken once a day and containing a proportionally higher content of active ingredient. The time-release effect can be obtained by capsule materials that dissolve at different pH values, by capsules that release slowly by osmotic pressure, or by any other known means of controlled release.

It can be necessary to use dosages outside these ranges in some cases as will be apparent to those skilled in the art. Further, it is noted that the clinician or treating physician will know how and when to start, interrupt, adjust, or terminate therapy in conjunction with individual patient response.

For the compositions and methods provided above, one of skill in the art will understand that preferred compounds for use in each are those compounds that are noted as preferred above. Still further preferred compounds for the compositions and methods are those compounds provided in the examples below.

**EXPERIMENTAL PART**

Hereinafter, the term "m.p." means melting point, "aq." means aqueous, "r.m." means reaction mixture, "r.t." means room temperature, 'DIPEA' means *N,N*-diisopropylethylamine, "DIPE" means diisopropylether, 'THF' means tetrahydrofuran, 'DMF' means dimethylformamide, 'DCM' means dichloromethane, "EtOH" means ethanol 'EtOAc' means ethylacetate, "AcOH" means acetic acid, "iPrOH" means isopropanol, "$iPrNH_2$" means isopropylamine, "MeCN" means acetonitrile, "MeOH" means methanol, "$Pd(OAc)_2$" means palladium(II)diacetate, "rac" means racemic, 'sat.' means saturated, 'SFC' means supercritical fluid chromatography, 'SFC-MS' means supercritical fluid chromatography/mass spectrometry, "LC-MS" means liquid chromatography/mass spectrometry, "GCMS" means gas chromatography/mass spectrometry, "HPLC" means high-performance liquid chromatography, "RP" means reversed phase, "UPLC" means ultra-performance liquid chromatography, "$R_t$" means retention time (in minutes), "$[M+H]^+$" means the protonated mass of the free base of the compound, "DAST" means diethylaminosulfur trifluoride, "DMTMM" means 4-(4,6-dimethoxy-1,3,5-triazin-2-yl)-4-methylmorpholinium chloride, "HATU" means *O*-(7-azabenzotriazol-1-yl)-*N,N,N',N'*-tetramethyluronium hexafluorophosphate, "Xantphos" means. (9,9-dimethyl-9H-xanthene-4,5-diyl)bis[diphenylphosphine], "TBAT" means tetrabutyl ammonium triphenyldifluorosilicate, "TFA" means trifuoroacetic acid, "$Et_2O$" means diethylether, "DMSO" means dimethylsulfoxide, "MeCN" means acetonitrile.

For key intermediates, as well as some final compounds, the absolute configuration of chiral centers (indicated as R and/or *S*) were established via comparison with samples of known configuration, or the use of analytical techniques suitable for the determination of absolute configuration, such as VCD (vibrational cicular dichroism) or X-ray crystallography. When the absolute configuration at a chiral center is unknown, it is arbitrarily designated R*.

<u>A. Preparation of the intermediates</u>

<u>Example A1</u>

**<u>Preparation of intermediate 1</u>.**

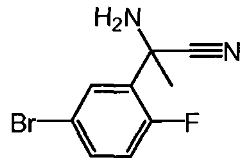

Trimethylsilylcyanide (30.7 mL, 230 mmol) was added to a stirred solution of 5-bromo-2-fluoroacetophenone (25 g, 115 mmol) and $NH_4Cl$ (18.5 g, 345 mmol) in $NH_3$/MeOH (150 mL). The mixture was stirred at room temperature for 3 days. Then the solvent was *evaporated in vacuo* and the residue was taken up in EtOAc (80 mL). The solid was filtered and the filtrate was evaporated *in vacuo* to yield **intermediate 1** (27.9 g, quant. yield) which was used in the next step without further purification.

Example A2

**Preparation of intermediate 2.**

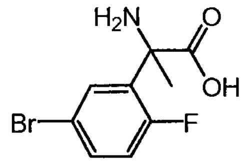

**Intermediate 1** (27 g, 111 mmol) was dissolved in HCl (37% in $H_2O$) (130 mL) and acetic acid (130 mL) and the mixture was refluxed for 16 hours. After cooling to room temperature, the mixture was concentrated *in vacuo.* Water was added and the aqueous layer was extracted with EtOAc. The aqueous layer was basified with aq. NaOH solution (25%) to pH 7. The aqueous layer was partially *concentrated in vacuo.* The mixture was cooled down in an ice bath and the precipitate was filtered off, washed with water and then $Et_2O$ and dried *in vacuo* to yield **intermediate 2** (18 g, 62% yield) as a white solid.

Example A3

**Preparation of intermediate 3.**

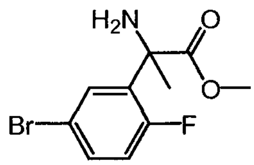

**Intermediate 2** (15 g, 57.2 mmol) was dissolved in MeOH (300 mL). $H_2SO_4$ (330 mL) was added and the reaction mixture was refluxed for 48 h. The r.m. was concentrated *in vacuo.* Water was added and the solution was basified to pH 8 with sat. aq. $NHCO_3$ solution. The aqueous layer was then extracted with EtOAc. The organic layer was separated, dried ($MgSO_4$), filtered and *concentrated in vacuo* to yield **intermediate 3** (15 g, 95% yield).

Example A4

Preparation of **intermediate 4**.

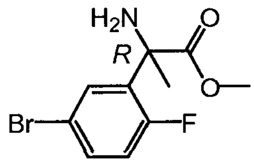

**Intermediate 3** (10 g) was separated into the corresponding enantiomers by preparative SFC on (Chiralpak® Daicel AD 30 x 250 mm). Mobile phase ($CO_2$, MeOH with 0.2% $iPrNH_2$) to yield **intermediate 4** (4.2 g, 42% yield). $\alpha_D$: -10.1° (365 nm, c 0.762 w/v %, MeOH, 20 °C).

Example A5

Preparation of **intermediate 5.**

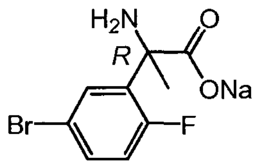

THF (150 mL) was added to a solution of **intermediate 4** (40 g, 145 mmol) in NaOH (1 M in $H_2O$, 360 mL). The mixture was stirred at r.t. for 4 hours. The mixture was *concentrated in vacuo* to afford **intermediate 5** (42 g) as a white solid, which was used as such in the next reaction step.

Example A6

Preparation of **intermediate 6**.

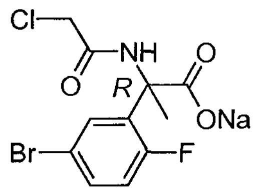

To a cooled solution of **intermediate 5** (41.3 g, 145 mmol) in $H_2O$ (150 mL), a solution of chloroacetyl chloride (24 mL, 304.5 mmol) in 1,4-dioxane (75 mL) was added dropwise. Simultaneously, NaOH (5M in $H_2O$, 29 mL) was added to adjust the pH at 10-11. The organic layer was separated, and the aqueous layer extracted with $Et_2O$. Then the aqueous layer was acidified with HCl (6 M, in $H_2O$) until pH 2. The precipitated white solid was collected by filtration, washed with $H_2O$ and dried to yield **intermediate 6** (42 g, 86% yield).

Example A7

Preparation of **intermediate 7.**

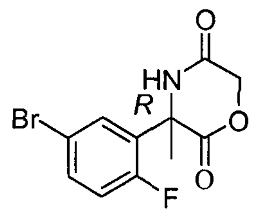

**Intermediate 6** (42 g, 124 mmol) and $NaHCO_3$ (20.8 g, 248 mmol) were dissolved in DMF (1000 mL), and the reaction mixture was stirred at 80 °C for 3 hours. The mixture was partially concentrated under reduced pressure, cooled to r.t. and then filtered over diatomaceous earth. The filtrate was concentrated *in vacuo,* and the residue was purified by flash column chromatography (silica gel; eluent: MeOH/DCM 0/100 to 5/95). The desired fractions were collected and concentrated *in vacuo* to yield **intermediate 7** (36 g, 96% yield).

Example A8

Preparation of **intermediate 8.**

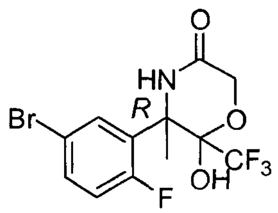

To a solution of **intermediate 7** (11.6 g, 38.5 mmol) in THF (117 mL) was added TBAT (2.08 g, 3.85 mmol). Then, (trifluoromethyl)trimethyl silane (12.5 mL, 84.6 mmol) was added dropwise, and the r.m. was stirred at r.t. for 20 minutes. The mixture was quenched with aqueous NaCl and extracted with EtOAc. The combined organic layers were dried ($MgSO_4$), filtered and concentrated *in vacuo* to yield **intermediate 8** (14 g, 98% yield) as a mixture of cis and trans isomers, which was used as such in the next step.

Example A9

Preparation of **intermediate 9.**

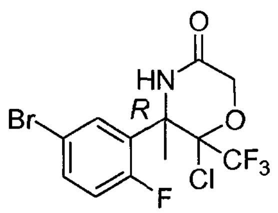

**Intermediate 8** (14 g, 37.6 mmol) was dissolved in DCM (600 mL) and cooled down to 0 °C Then thionyl chloride (11.2 mL, 150 mmol) was added dropwise. The reaction mixture was stirred for 30 min at 0 °C and then pyridine (18.2 mL, 225.7 mmol) was added. After 30 minutes the reaction was hydrolyzed with an aqueous 1N HCl solution and then extracted with DCM. The organic layers were separated, dried ($MgSO_4$), filtered and evaporated *in vacuo.* The crude product was purified by flash column chromatography (silica gel; eluent: 7 M solution of ammonia in methanol/DCM 0/100 to 2/98). The desired fractions were collected and concentrated *in vacuo* to yield **intermediate 9** (6 g, 41% yield, mixture of diastereoisomers).

Example A10

Preparation of **intermediate 10.**

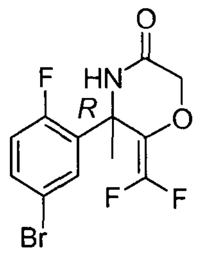

**Intermediate 9** (7 g, 17.9 mmol) and zinc copper couple (8.55 g, 66.3 mmol) were stirred in acetic acid (420 mL) at r.t. for 16 hours. The reaction mixture was filtered, washed with DCM and concentrated *in vacuo.* Ammonium hydroxide solution (28% in water) and DCM were added and the mixture was stirred at r.t. for 1 h. The organic layer was separated and the aqueous layer was extracted with DCM. The combined organic layers were dried ($MgSO_4$), filtered and *evaporated in vacuo* to yield **intermediate 10** (6 g, 99% yield) as a white powder.

Example A11

Preparation of **intermediate 11.**

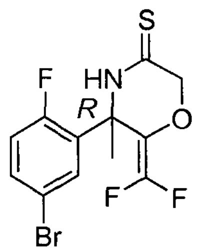

$P_2S_5$ (5.95 g, 26.8 mmol) was added to a solution of **intermediate 10** (6 g, 17.9 mmol) in THF (145 mL) at room temperature. The reaction mixture was stirred at 70 °C for 90 minutes. Then the mixture was cooled down to r.t., filtered off and the organic solvent evaporated *in vacuo* to yield **intermediate 11** (5.9 g), which was used as such in the next step.

Example A12

Preparation of **intermediate 12.**

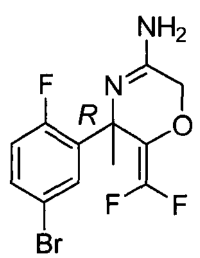

**Intermediate 11** (5.9 g, 16.8 mmol) was dissolved in 7N ammonia in MeOH (390 mL) and the reaction mixture was stirred at 80 °C for 2 hours. The solvent was evaporated and the crude product purified by column chromatography

(silica gel; eluent: 7 M solution of ammonia in methanol/DCM 0/100 to 5/95). The desired fractions were collected and concentrated *in vacuo* to yield **intermediate 12** (4.04 g, 72% yield).

Example A13

Preparation of **intermediate 13.**

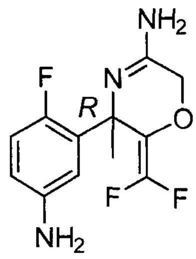

**Intermediate 12** (3.6 g, 10.7 mmol) was combined with $NaN_3$ (1.75 g, 26.9 mmol), CuI (2.56 g, 13.4 mmol) and $Na_2CO_3$ (2.28 g, 21.5 mmol) in DMSO (153 mL) and the reaction was degassed. After that, *N,N'*-dimethylethylenediamine (2 mL, 18.8 mmol) was added and the mixture was heated at 110 °C until completion of the reaction, about 3 hours. The reaction mixture was concentrated *in vacuo.* 7N ammonia in MeOH was added and the mixture was stirred overnight. The precipitate formed was filtered off and the filtrate was concentrated *in vacuo.* The crude product was purified by column chromatography (silica gel; eluent: 7 M solution of ammonia in methanol/DCM 0/100 to 30/70). The desired fractions were collected and *concentrated in vacuo* to yield **intermediate 13** (1.52 g, 52% yield).

Example A14

Preparation of **intermediate 14.**

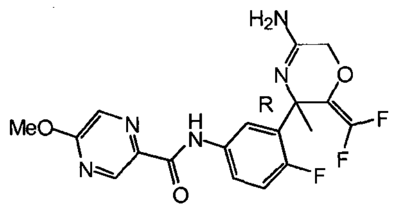

5-Methoxypyrazine-2-carboxylic acid (0.218 g, 1.42 mmol) was dissolved in MeOH (30 mL) and DMTMM (0.456 g, 1.548 mmol) was added. After stirring the mixture for 5 minutes, a solution of **intermediate 13** (0.35 g, 1.29 mmol) in MeOH (20 mL) was added at 0 °C, and the mixture was stirred for 16 h at room temperature. The solvent was evaporated *in vacuo.* The crude material was purified by flash column chromatography (silica gel; eluent: 7 M solution of ammonia in methanol/DCM 0/100 to 5/95). The desired fractions were collected and concentrated *in* vacuo. The residue was suspended from DIPE/heptanes, filtered and dried under high vacuum to yield **intermediate 14** (0.266 g, 51% yield) as a white solid.

Example A15

Preparation of **intermediate 15.**

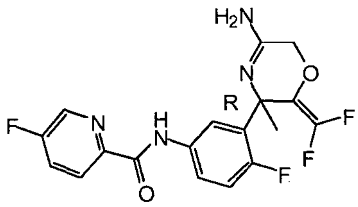

**Intermediate 15** was synthesized following the same approach described in the Example A14. Starting from **intermediate 13** (0.35 g, 1.29 mmol) **intermediate 15** was obtained as white solid (0.362 g, 71% yield).

Example A16

Preparation of **intermediate 16.**

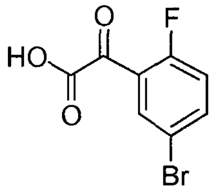

1-(5-bromo-2-fluoro-phenyl)ethanone [(CAS 198477-89-3), 70 g, 322 mmol) and selenium oxide (71.6 g, 645 mmol) were dissolved in pyridine (520 mL). The reaction mixture was stirred at 100 °C for 2 hours. The solvent was evaporated and aqueous HCl 1N solution was added. The aqueous layer was extracted with EtOAc. The combined organic layers were dried ($Mg_2SO_4$), filtered and concentrated *in vacuo* to yield **intermediate 16** (62 g, 78% yield), which was used as such in the next reaction.

Example A17

Preparation of **intermediate 17.**

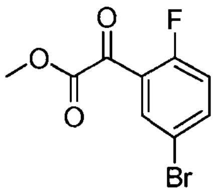

Thionyl chloride (37 mL, 510 mmol) was added dropwise to a stirred solution of **intermediate 16** (42 g, 170 mmol) in MeOH (456 mL) at 0 °C. The mixture was refluxed for 18 hours. The solvents were *evaporated in vacuo* and the residue was partitioned between saturated $Na_2CO_3$ and DCM. The organic layer was separated, dried ($Mg_2SO_4$), filtered and concentrated *in vacuo* to yield **intermediate 17** (30 g, 68% yield) as a yellow oil.

Example A18

Preparation of **intermediate 18.**

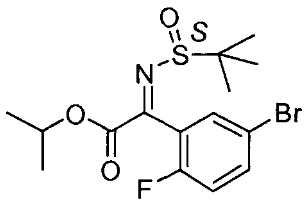

Titanium(IV) isopropoxide (153 mL, 522 mmol) was added to a stirred mixture of **intermediate 17** (68 g, 261 mmol) and (*S*)-2-methyl-2-propanesulfinamide (37.9 g, 313 mmol) in n-heptane (1000 mL). The mixture was stirred at 80 °C for 1.5 hours. The mixture was cooled down to r.t., and ice-water was added. The resulting mixture was filtered over a diatomaceous earth pad and rinsed with n-heptane. The aqueous layer was extracted with EtOAc. The combined organic layers were dried ($MgSO_4$), filtered and concentrated *in vacuo* to yield **intermediate 18** (87.9 g, 86% yield), which was used as such in the next reaction.

Example A19

Preparation of **intermediate 19.**

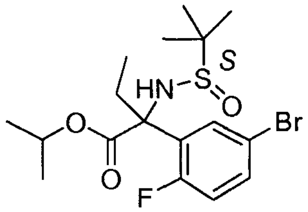

Ethylmagnesium bromide (3 M, 86 mL, 259 mmol) was added dropwise to a stirred solution of **intermediate 18** (72.6 g, 185 mmol) in DCM (1154 mL) at -78 °C under nitrogen. The mixture was stirred at this temperature for 30 min, and then the reaction was quenched by the addition of a sat. aq. NH4Cl solution, followed by water. The mixture was extracted with DCM and washed with water. The organic layer was separated, dried ($MgSO_4$), filtered and the solvents evaporated *in vacuo.* The crude product was purified by flash column chromatography (silica gel; eluent: Heptanes/EtOAc 90/10 to 70/30). The desired fractions were collected and *concentrated in vacuo* to yield **intermediate 19** (25.56 g, 33% yield, mixture of diastereomers) as a yellow oil.

Example A20

Preparation of **intermediate 20.**

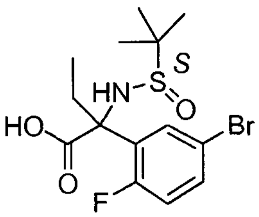

A 2M aq. NaOH solution (91 mL, 181.8 mmol) was added to a solution of crude **intermediate 19** (25.6 g, 60.6 mmol) in MeOH (68 mL). The resulting mixture was stirred at reflux for 5 hours. The mixture was cooled to r.t., and then partitioned between water and EtOAc. The aqueous layer was separated and neutralized by the addition of a 1M aq. HCl solution, and then extracted with DCM. The organic layer was separated, dried (MgSO4), filtered and the solvents evaporated *in vacuo.* The residue was suspended from DIPE and the precipitate was filtered off and dried *in vacuo* to yield **intermediate 20** (17.5 g, 76% yield, mixture of diastereomers) as a white solid.

Example A21

Preparation of **intermediate 21.**

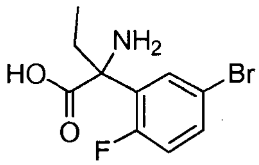

Hydrochloric acid salt

**Intermediate 20** (17.5 g, 46 mmol) was stirred in 4M HCl solution in dioxane (46 mL) at room temperature for 15 min. To the resulting suspension, DIPE was added, and the precipitate was filtered off and dried *in vacuo* to yield **intermediate 21** (15.1 g, quant. yield, racemate) as a white solid.

Example A22

Preparation of **intermediate 22.**

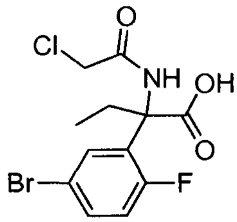

To a cooled solution of **intermediate 21** (15.1 g, 43.2 mmol) and DIPEA (35 mL, 203.7 mmol) in DCM (350 mL), chloroacetyl chloride (5.6 mL, 70.6 mmol) was added dropwise at 0 °C. After stirring at 0 °C for 15 minutes, the reaction mixture was warmed to rt and acidified with HCl (2 M in $H_2O$, 10 mL). The mixture was extracted with EtOAc and washed with brine. The organic layer was separated, dried (MgSO4), filtered and the solvents evaporated *in vacuo.* The crude product was triturated with DIPE and the precipitate was filtered off and dried *in vacuo* to yield **intermediate 22** (8.04 g, 53% yield) as a brown solid.

Example A23

Preparation of **intermediate 23.**

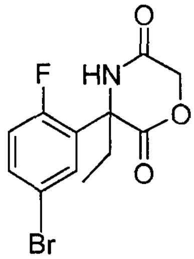

**Intermediate 23** was synthesized following the same approach described in the Example 7. Starting from **intermediate 22** (8 g, 22.69 mmol) **intermediate 23** was obtained as a white solid (4.5 g, 63% yield).

Example A24

Preparation of **intermediate 24.**

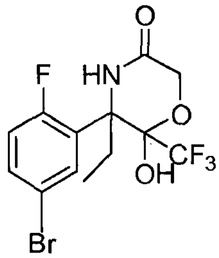

**Intermediate 24** was synthesized following the same approach described in the Example 8. Starting from **intermediate 23** (2.34 g, 7.4 mmol) **intermediate 24** was obtained as an oil (1.9 g, 66% yield).

Example A25

Preparation of **intermediate 25.**

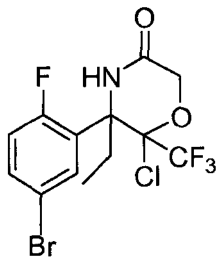

**Intermediate 25** was synthesized following the same approach described in the Example 9. Starting from **intermediate 24** (1.9 g, 4.92 mmol) **intermediate 25** was obtained as a pale yellow solid (1.58 g, 79% yield).

Example A26

Preparation of **intermediate 26.**

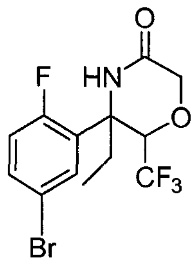

**Intermediate 25** (1.4 g, 3.46 mmol) was stirred in acetic acid (42 mL) at 100 °C for 5 minutes. Zinc (0.91 g, 13.8 mmol) was added and the mixture was stirred at 100 °C for 1 h. Extra zinc (0.452 g, 6.9 mmol) was added and the mixture was further stirred at 100 °C. After another hour, new zinc (0.226 g, 3.46 mmol) was added and the mixture was stirred at 100 °C for 2 h. Finally, extra zinc (0.91 g, 13.8 mmol) was added and the mixture was stirred at 100 °C for 1

h. After cooling, the reaction mixture was filtered, washed with DCM and concentrated *in vacuo.* Ammonium hydroxide solution (28% in water), aq. sat. $NaHCO_3$ solution and water were added. The aqueous layer was extracted with DCM. The combined organic layers were dried ($MgSO_4$), filtered and evaporated *in vacuo* to yield **intermediate 26** (1.26 g, 98% yield) as a white solid.

Example A27

Preparation of **intermediate 27.**

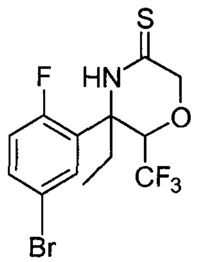

**Intermediate 27** was synthesized following the same approach described in the Example 11. Starting from **intermediate 26** (1.26 g, 3.4 mmol) **intermediate 27** was obtained as a white solid (1.09 g, 83% yield).

Example A28

Preparation of **intermediate 28 and intermediate 29.**

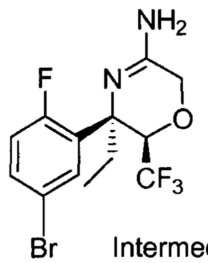

Intermediate 28

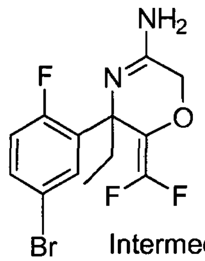

Intermediate 29

**Intermediate 27** (1 g, 2.59 mmol) was dissolved in 7N ammonia in MeOH (60 mL) and the reaction mixture was stirred at 130 °C for 15 minutes under microwave radiation. The reaction mixture was *concentrated in vacuo* and new 7N ammonia in MeOH (30 mL) was added. The reaction mixture was stirred at 130 °C for another 15 minutes under microwave radiation. The solvent was evaporated and the crude product purified by column chromatography (silica gel; eluent: 7 M solution of ammonia in methanol/DCM 0/100 to 2/98). The desired fractions were collected and concentrated *in vacuo* to yield **intermediate 28** (0.29 g, 30% yield, *cis* racemate) as a white solid and **intermediate 29** (0.27 g, 30% yield) as an oil.

Example A29

Preparation of **intermediate 30.**

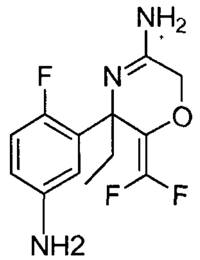

**Intermediate 30** was synthesized following the same approach described in the Example 13. Starting from **intermediate 29** (0.189 g, 0.542 mmol) **intermediate 30** was obtained as an oil (0.16 g), which was used as such in the next reaction.

Example A30

Preparation of **intermediate 31.**

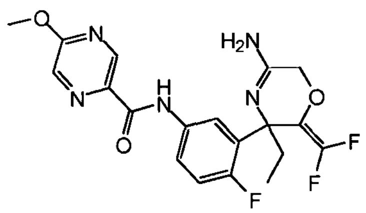

**Intermediate 31** was synthesized following the same approach described in the Example A14. Starting from **intermediate 30** (0.09 g, 0.315 mmol) **intermediate 31** was obtained as an off white solid (0.028 g, 21% yield).

Example A31

Preparation of **intermediate 32.**

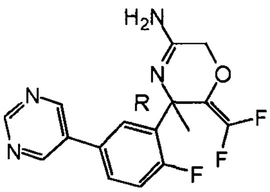

**Intermediate 12** (0.4 g, 1.194 mmol), 5-pyrimidinylboronic acid (0.296 g, 2.387 mmol) and tetrakis(triphenylphosphine) palladium(0) (0.207 g, 0.179 mmol) were dissolved in a mixture of 1,4-dioxane (18 mL) and aqueous $NaHCO_3$ (sat. sol., 8.5 mL). The resulting mixture was flushed with $N_2$ and then heated at 70 °C for 2 hours. The reaction mixture was then diluted with water and then extracted with DCM (3x). The combined organic layer was washed with brine, dried ($Na_2SO_4$), filtered and the solvents evaporated *in vacuo.* The crude product was purified by flash column chromatography (silica gel; 7 M solution of ammonia in methanol/DCM 0/100 to 5/95). The desired fractions were collected and *concentrated in vacuo* to yield **intermediate 32** (0.3 g, 75% yield) as a white foam.

Example A32

Preparation of **intermediate 33.**

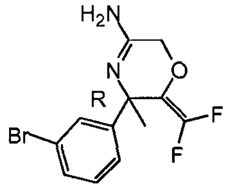

Starting from 3-bromoacetophenone (CAS 2142-63-4), **intermediate 33** was synthesized following the same reaction procedures as described for intermediate 12 in Examples A1-A12.

Example A33

Preparation of **intermediate 34.**

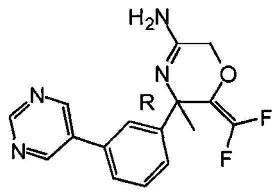

**Intermediate 34** was synthesized following the same approach described in the Example A31. Starting from **intermediate 33** (0.31 g, 0.978 mmol) **intermediate 34** was obtained as a white solid (0.21 g, 68% yield).

Example A34

Preparation of **intermediate 35.**

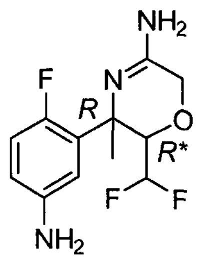

**Intermediate 13** (2.78 g, 10.25 mmol) was dissolved in EtOAc (70 mL) and palladium on carbon (10%) (1.09 g) and thiophene (0.4% solution in THF, 14 mL) were added. The mixture was hydrogenated at rt and atmospheric pressure for 16 hours. The catalyst was filtered off and the solvents evaporated *in vacuo.* The crude product was purified by flash column chromatography (silica gel; eluent: 7 M solution of ammonia in methanol/DCM 0/100 to 10/90). The desired fractions were collected and concentrated *in vacuo* to yield **intermediate 35** (0.478 g, 17% yield)

B. Preparation of the final compounds

Example B1

Preparation of **compound 1:** *N*-{3-[(2R*,3R)-5-Amino-2-(difluoromethyl)-3-methyl-3,6-dihydro-2H-1,4-oxazin-3-yl]-4-fluorophenyl}-5-methoxypyrazine-2-carboxamide

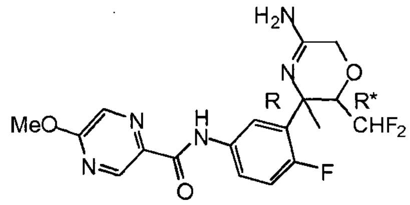

**Intermediate 14** (0.154 g, 0.378 mmol) was dissolved in EtOAc (5 mL) and palladium on carbon (10%) (0.04 g, 0.038 mmol) and thiophene (0.4% solution in THF, 0.5 mL, 0.026 mmol) were added. The mixture was hydrogenated at rt and atmospheric pressure for 16 hours. The catalyst was filtered off and the solvents *evaporated in vacuo.* The crude product was purified by flash column chromatography (silica gel; eluent: 7 M solution of ammonia in methanol/DCM 0/100 to 2/98). The desired fractions were collected and *concentrated in vacuo.* The residue was suspended from DIPE, filtered and dried under high vacuum to yield **compound 1** (0.067 g, 43% yield).

Example B2

Preparation of **compound 2:** *N*-{3-[(2R*,3R)-5-Amino-2-(difluoromethyl)-3-methyl-3,6-dihydro-2H-1,4-oxazin-3-yl]-4-fluorophenyl}-5-fluoropyridine-2-carboxamide

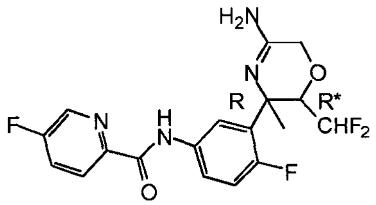

**Compound 2** was synthesized following the same approach described in the Example B1. Starting from **intermediate 15** (0.251 g, 0.637 mmol) **compound 2** was obtained as white solid (0.114 g, 45% yield).

Example B3

Preparation of **compound 3:** *cis*-rac-*N*-{3-[5-Amino-2-(difluoromethyl)-3-ethyl-3,6-dihydro-2H-1,4-oxazin-3-yl]-4-fluorophenyl}-5-methoxypyrazine-2-carboxamide

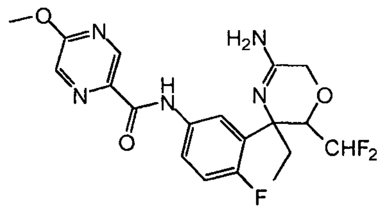

**Intermediate 31** (0.028 g, 0.066 mmol) was dissolved in MeOH (1.3 mL) and hydrogenated in a H-cube reactor (1 mL/min, 10% Pd/C cartridge, full H2 mode), first at 25 °C, then at 50 °C and finally at 80 °C. The solvent was *evaporated in vacuo.* The crude product was purified by preparative HPLC (C18 XBridge 19 x 100 5 um), mobile phase (gradient from 80% 0.1 % $NH_4CO_3H/NH_4OH$ pH 9 solution in Water, 20% $CH_3CN$ to 0% 0.1% $NH_4CO_3H/NH_4OH$ pH 9 solution in Water, 100% $CH_3CN$). The desired fractions were collected and *concentrated in vacuo* to yield **compound 3** (0.0032 g, 11% yield).

Example B4

Preparation of **compound 4:** (5R,6R*)-6-(Difluoromethyl)-5-(2-fluoro-5-pyrimidin-5-ylphenyl)-5-methyl-5,6-dihydro-2H-1,4-oxazin-3-amine

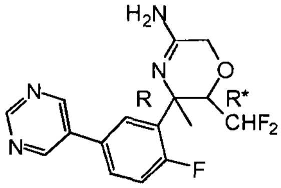

**Compound 4** was synthesized following the same approach described in the Example B1. Starting from **intermediate 32** (0.155 g, 0.464 mmol) **compound 4** was obtained (0.018 g, 12% yield).

Example B5

Preparation of **compound 5**: (5R,6R*)-6-(Difluoromethyl)-5-methyl-5-(3-pyrimidin-5-ylphenyl)-5,6-dihydro-2H-1,4-oxazin-3-amine

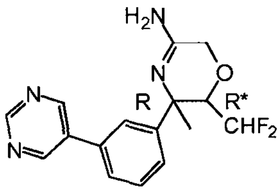

**Compound 5** was synthesized following the same approach described in the Example B1. Starting from **intermediate 34** (0.124 g, 0.392 mmol) **compound 5** was obtained as a white solid (0.04 g, 32% yield).

Example B6

Preparation of **compound 6**: *N*-{3-[(2R*,3R)-5-Amino-2-(difluoromethyl)-3-methyl-3,6-dihydro-2H-1,4-oxazin-3-yl]-4-fluorophenyl}-5-chloropyridine-2-carboxamide

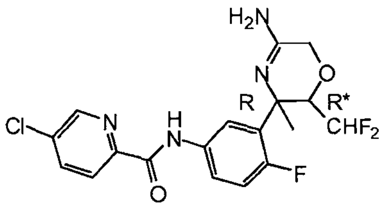

5-Chloropyridine-2-carboxylic acid (63 mg, 0.4 mmol) was dissolved in MeOH (7 mL) and DMTMM (129 mg, 0.44 mmol) was added. After stirring the mixture for 5 minutes, a solution of **intermediate 35** (100 mg, 0.366 mmol) in MeOH (8 mL) was added at 0 °C, and the mixture was stirred for 16 h at room temperature. The solvent was *evaporated in vacuo.* The crude material was purified by flash column chromatography (silica gel; eluent: 7 M solution of ammonia in methanol/DCM 0/100 to 5/95). The desired fractions were collected and concentrated *in* vacuo. The residue was triturated with DIPE, filtered and dried under high vacuum to yield **compound 6** (0.116 g, 74% yield) as a white solid

Example B7

Preparation of **compound 7**: *N*-{3-[(2R*,3R)-5-Amino-2-(difluoromethyl)-3-methyl-3,6-dihydro-2H-1,4-oxazin-3-yl]-4-fluorophenyl}-5-cyanopyridine-2-carboxamide

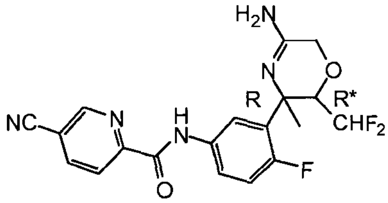

**Compound 7** was synthesized following the same approach described in the Example B6. Starting from **intermediate 35**(100 mg, 0.4 mmol) **compound 7** was obtained (110 mg, 75% yield).

Compounds 1 to 7 in table1 list the compounds that were prepared according to one of the above Examples. 'Ex. No.' refers to the Example number according to which protocol the compound was synthesized. 'Co. No.' means compound number. $C_2$(R*) means that the absolute configuration at $C_2$ is either R or S but unknown yet.

**Table 1:**

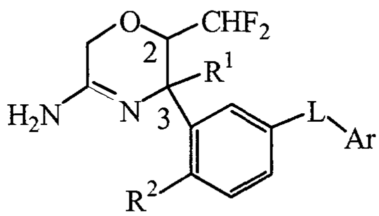

| Co. No. | Ex. No. | $R^1$ | $R^2$ | ---L-Ar | Stereochemistry |
|---|---|---|---|---|---|
| **1** | B1 | $CH_3$ | F | 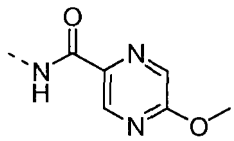 | $C_2$(R*);$C_3$(R) Single diastereoisomer Pure enantiomer |
| **2** | B2 | $CH_3$ | F | 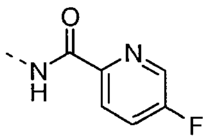 | $C_2$(R*);$C_3$(R) Single diastereoisomer Pure enantiomer |
| **3** | B3 | $CH_2CH_3$ | F | 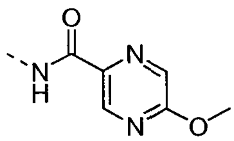 | $C_2$(RS);$C_3$(RS) Single diastereoisomer (Cis) |
| **4** | B4 | $CH_3$ | H | 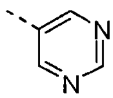 | $C_2$(R*);$C_3$(R) Single diastereoisomer Pure enantiomer |
| **5** | B5 | $CH_3$ | F | 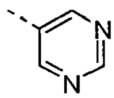 | $C_2$(R*);$C_3$(R) Single diastereoisomer Pure enantiomer |

(continued)

| Co. No. | Ex. No. | $R^1$ | $R^2$ | ---L-Ar | Stereochemistry |
|---|---|---|---|---|---|
| **6** | B6 | $CH_3$ | F | 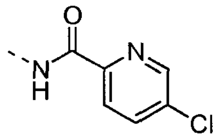 | $C_2(R^*);C_3(R)$ Single diastereoisomer Pure enantiomer |
| 7 | B7 | $CH_3$ | F | 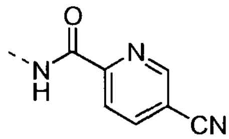 | $C_2(R^*);C_3(R)$ Single diastereoisomer Pure enantiomer |

C. Analytical Part

**LCMS**

For (LC)MS-characterization of the compounds of the present invention, the following methods were used.

*Method 1 :*

The LC measurement was performed using an Acquity UPLC (Waters) system comprising a binary pump, a sample organizer, a column heater (set at 55°C), a diode-array detector (DAD) and a column as specified in the respective methods below. Flow from the column was split to a MS spectrometer. The MS detector was configured with an electrospray ionization source. Mass spectra were acquired by scanning from 100 to 1000 in 0.18 seconds using a dwell time of 0.02 seconds. The capillary needle voltage was 3.5 kV and the source temperature was maintained at 140°C. Nitrogen was used as the nebulizer gas. Data acquisition was performed with a Waters-Micromass MassLynx-Openlynx data system.

Reversed phase UPLC (Ultra Performance Liquid Chromatography) was carried out on a bridged ethylsiloxane/silica hybrid (BEH) C18 column (1.7 μm, 2.1 x 50 mm; Waters Acquity) with a flow rate of 0.8 ml/min. Two mobile phases (10 mM ammonium acetate in $H_2O$/acetonitrile 95/5; mobile phase B: acetonitrile) were used to run a gradient condition from 95 % A and 5 % B to 5 % A and 95 % B in 1.3 minutes and hold for 0.7 minutes. An injection volume of 0.75 μl was used.

Cone voltage was 10 V for positive ionization mode and 20 V for negative ionization mode.

*Method 2:*

The UPLC (Ultra Performance Liquid Chromatography) measurement was performed using an Acquity UPLC (Waters) system comprising a sampler organizer, a binary pump with degasser, a four column's oven, a diode-array detector (DAD) and a column as specified in the respective methods. The MS detector was configured with an ESCI dual ionization source (electrospray combined with atmospheric pressure chemical ionization). Nitrogen was used as the nebulizer gas. The source temperature was maintained at 140 °C. Data acquisition was performed with MassLynx-Openlynx software.

Reversed phase UPLC (Ultra Performance Liquid Chromatography) was carried out on a RRHD Eclipse Plus-C18 (1.8 μm, 2.1 x 50 mm) from Agilent, with a flow rate of 1.0 ml/min, at 50°C without split to the MS detector. The gradient conditions used are: 95 % A (0.5 g/l ammonium acetate solution + 5 % acetonitrile), 5 % B (acetonitrile), to 40 % A, 60 % B in 3.8 minutes, to 5 % A, 95 % B in 4.6 minutes, kept till 5.0 minutes. Injection volume 2 μl. Low-resolution mass spectra (single quadrupole, SQD detector) were acquired by scanning from 100 to 1000 in 0.1 seconds using an inter-channel delay of 0.08 second. The capillary needle voltage was 3 kV. The cone voltage was 25 V for positive ionization mode and 30 V for negative ionization mode.

*Method 3:*

The LC measurement was performed using an Acquity UPLC (Waters) system comprising a binary pump, a sample organizer, a column heater (set at 55 °C), a diode-array detector (DAD) and a column as specified in the respective methods below. Flow from the column was split to a MS spectrometer. The MS detector was configured with an electrospray ionization source. Mass spectra were acquired by scanning from 100 to 1000 in 0.18 seconds using a dwell time of 0.02 seconds. The capillary needle voltage was 3.5 kV and the source temperature was maintained at 140 °C. Nitrogen was used as the nebulizer gas. Data acquisition was performed with a Waters-Micromass MassLynx-Openlynx data system.

Reversed phase UPLC (Ultra Performance Liquid Chromatography) was carried out on a bridged ethylsiloxane/silica hybrid (BEH) C18 column (1.7 μm, 2.1 x 50 mm; Waters Acquity) with a flow rate of 0.8 ml/min. Two mobile phases (10 mM ammonium acetate in $H_2O$/acetonitrile 95/5; mobile phase B: acetonitrile) were used to run a gradient condition from 95 % A and 5 % B to 5 % A and 95 % B in 1.3 minutes and hold for 0.3 minutes. An injection volume of 0.5 μl was used. Cone voltage was 10 V for positive ionization mode and 20 V for negative ionization mode.

**Melting Points**

Values are either peak values or melt ranges, and are obtained with experimental uncertainties that are commonly associated with this analytical method.

<u>DSC823e (indicated by DSC in Table 2)</u>

For a number of compounds, melting points were determined with a DSC823e (Mettler-Toledo). Melting points were measured with a temperature gradient of 30° C/minute. Maximum temperature was 400°C.

**<u>Table 2</u>: Analytical data** - $R_t$ means retention time (in minutes), $[M+H]^+$ means the protonated mass of the compound, method refers to the method used for (LC)MS.

| Co. Nr. | $R_t$ | $[M+H]^+$ | Method | Melting Point |
|---|---|---|---|---|
| **1** | 0.75 | 410 | 1 | 227.3°C |
| **2** | 0.76 | 397 | 1 | 205.7°C |
| **3** | 1.98 | 424 | 2 | n.d. |
| **4** | 0.59 | 337 | 1 | n.d. |
| **5** | 0.55 | 319 | 1 | n.d. |
| **6** | 0.89 | 413 | 3 | 203.9°C |
| **7** | 0.87 | 404 | 3 | 230.9°C |
| n.d. means not determined | | | | |

**Optical Rotations**

Optical rotations were measured on a Perkin-Elmer 341 polarimeter with a sodium lamp and reported as follows: $[\alpha]_{\lambda}^{t°C}$ (c g/100 ml, solvent).

**<u>Table 3:</u> Analytical data - Optical rotation values for enantiomerically pure compounds**

| Co. Nr. | $\alpha_D$(°) | Wavelength (nm) | Concentration w/v % | Solvent | Temp. (°C) |
|---|---|---|---|---|---|
| 2 | +121.95 | 365 | 0.205 | DMF | 20 |
| 7 | +11.85 | 589 | 0.27 | DMF | 20 |

**NMR**

For a number of compounds, $^1H$ NMR spectra were recorded on a Bruker DPX-360, on a Bruker DPX-400 or on a Bruker Avance 600 spectrometer with standard pulse sequences, operating at 360 MHz, 400 MHz and 600 MHz respectively, using CHLOROFORM-*d* (deuterated chloroform, $CDCl_3$) or DMSO-$d_6$ (deuterated DMSO, dimethyl-d6 sulfoxide) as solvents. Chemical shifts (δ) are reported in parts per million (ppm) relative to tetramethylsilane (TMS), which was used as internal standard.

Table 4:

| Co. Nr. | NMR result |
|---|---|
| **1** | $^1$H NMR (360 MHz, DMSO-$d_6$) δ ppm 1.52 (s, 3 H), 3.93 - 4.01 (m, 1 H), 4.02 (s, 3 H), 4.10 (d, *J*=15.9 Hz, 1 H), 4.15 (d, *J*=15.9 Hz, 1 H), 5.64 (td, *J*=54.0, 4.4 Hz, 1 H), 5.80 (br. s, 2 H), 7.11 (dd, *J*=12.1, 8.8 Hz, 1 H), 7.80 (ddd, *J*=8.8, 4.1, 2.7 Hz, 1 H), 8.04 (dd, *J*=7.3, 2.8 Hz, 1 H), 8.42 (d, *J*=1.3 Hz, 1 H), 8.88 (d, *J*=1.3 Hz, 1 H), 10.44 (s, 1 H). |
| **2** | $^1$H NMR (360 MHz, DMSO-$d_6$) δ ppm 1.52 (s, 3 H), 3.94 - 4.05 (m, 1 H), 4.10 (d, *J*=16.0 Hz, 1 H), 4.15 (d, *J*=16.0 Hz, 1 H), 5.65 (td, *J*=54.3, 4.3 Hz, 1 H), 5.81 (br. s, 2 H), 7.11 (dd, *J*=12.1, 8.8 Hz, 1 H), 7.82 (dt, *J*=8.5, 3.6 Hz, 1 H), 7.97 (dd, *J*=8.8, 2.9 Hz, 1 H), 8.03 (td, *J*=6.9, 2.7 Hz, 1 H), 8.22 (dd, *J*=8.8, 4.8 Hz, 1 H), 8.74 (d, *J*=2.9 Hz, 1 H), 10.55 (br. s, 1 H). |
| **3** | H$^1$ NMR (400 MHz, CHLOROFORM-*d*) δ ppm 0.77 (t, *J*=7.4 Hz, 3 H), 1.97 - 2.07 (m, 1 H), 2.13 - 2.26 (m, 1 H), 4.07 (s, 3 H), 4.32 (br. s., 2 H), 4.18 (dd, *J*=15.7, 1.2 Hz, 1 H), 4.33 (dd, *J*=15.6, 1.5 Hz, 1 H), 4.36 (ddt, *J*=21.0, 8.4,2.2,2.2 Hz, 1 H), 5.46 (td, *J*=54.0, 2.6 Hz, 1 H), 7.07 (dd, *J*=11.6, 8.8 Hz, 1 H), 7.83 (dd, *J*=6.7, 2.8 Hz, 1 H), 8.07 (ddd, *J*=8.8, 4.2, 2.9 Hz, 1 H), 8.16 (d, *J*=1.4 Hz, 1 H), 9.02 (d, *J*=1.2 Hz, 1 H), 9.56 (br. s, 1 H). |
| **4** | $^1$H NMR (360 MHz, DMSO-$d_6$) δ ppm 1.56 (s, 3 H), 4.05 - 4.13 (m, 1 H), 4.11 - 4.17 (m, 2 H), 5.80 (td, *J*=53.8, 4.0 Hz, 1 H), 5.90 (br. s., 2 H), 7.31 (dd, *J*=12.1, 8.4 Hz, 1 H), 7.76 (ddd, *J*=8.3, 4.5, 2.6 Hz, 1 H), 8.10 (dd, *J*=7.5, 2.4 Hz, 1 H), 9.08 (s, 2 H), 9.20 (s, 1 H). |
| **5** | $^1$H NMR (360 MHz, DMSO-$d_6$) δ ppm 1.57 (s, 3 H), 3.85 (ddd, *J*=15.2, 7.5, 4.8 Hz, 2 H), 4.11 (d, *J*=15.7 Hz, 1 H), 4.26 (d, *J*=15.7 Hz, 1 H), 5.24 - 5.64 (m, 1 H), 5.78 (br. s., 2 H), 7.43 - 7.53 (m, 2 H), 7.64 - 7.74 (m, 1 H), 9.10 (s, 2 H), 9.20 (s, 1 H). |
| **6** | $^1$H NMR (360 MHz, DMSO-$d_6$) δ ppm 1.53 (s, 3 H) 4.00 (ddd, *J*=14.2, 10.0, 4.0 Hz, 1 H) 4.07 - 4.22 (m, 2 H) 5.47 - 5.81 (m, 1 H) 5.82 (s, 2 H) 7.12 (dd, *J*=12.1, 8.8 Hz, 1 H) 7.78 - 7.88 (m, 1 H) 8.04 (dd, *J*=7.1, 2.7 Hz, 1 H) 8.11 - 8.18 (m, 1 H) 8.20 (dd, *J*=8.4, 2.6 Hz, 1 H) 8.79 (d, *J*=1.8 Hz, 1 H) 10.61 (s, 1 H) |
| **7** | $^1$H NMR (360 MHz, DMSO-$d_6$) δ ppm 1.53 (s, 3 H) 3.90 - 4.06 (m, 1 H) 4.06 - 4.21 (m, 2 H) 5.47 - 5.81 (m, 1 H) 5.81 (br. s., 2 H) 7.13 (dd, *J*=12.1, 8.8 Hz, 1 H) 7.76 - 7.90 (m, 1 H) 8.07 (dd, *J*=7.3, 2.9 Hz, 1 H) 8.28 (d, *J*=8.4 Hz, 1 H) 8.58 (dd, *J*=8.2, 2.0 Hz, 1 H) 9.20 (d, *J*=1.8 Hz, 1 H) 10.78 (s, 1 H) |

D. Pharmacological examples

The compounds provided in the present invention are inhibitors of the beta-site APP-cleaving enzyme 1 (BACE1). Inhibition of BACE1, an aspartic protease, is believed to be relevant for treatment of Alzheimer's Disease (AD). The production and accumulation of beta-amyloid peptides (Abeta) from the beta-amyloid precursor protein (APP) is believed to play a key role in the onset and progression of AD. Abeta is produced from the amyloid precursor protein (APP) by sequential cleavage at the N- and C-termini of the Abeta domain by beta-secretase and gamma-secretase, respectively.

Compounds of Formula (I) are expected to have their effect substantially at BACE1 by virtue of their ability to inhibit the enzymatic activity. The behaviour of such inhibitors tested using a biochemical Fluorescence Resonance Energy Transfer (FRET) based assay and a cellular αLisa assay in SKNBE2 cells described below and which are suitable for the identification of such compounds, and more particularly the compounds according to Formula (I), are shown in Table 5 and Table 6.

*Biochemical FRET based assay*

This assay is a Fluorescence Resonance Energy Transfer Assay (FRET) based assay. The substrate for this assay is an APP derived 13 amino acids peptide that contains the 'Swedish' Lys-Met/Asn-Leu mutation of the amyloid precursor protein (APP) beta-secretase cleavage site. This substrate also contains two fluorophores: (7-methoxycoumarin-4-yl) acetic acid (Mca) is a fluorescent donor with excitation wavelength at 320 nm and emission at 405 nm and 2,4-Dinitrophenyl (Dnp) is a proprietary quencher acceptor. The distance between those two groups has been selected so that upon light excitation, the donor fluorescence energy is significantly quenched by the acceptor, through resonance energy transfer. Upon cleavage by BACE1, the fluorophore Mca is separated from the quenching group Dnp, restoring the full fluorescence yield of the donor. The increase in fluorescence is linearly related to the rate of proteolysis.

Briefly in a 384-well format recombinant BACE1 protein in a final concentration of 1 μg/ml is incubated for 120 minutes at room temperature with 10 μm substrate in incubation buffer (40 mM Citrate buffer pH 5.0, 0.04 % PEG, 4 %

DMSO) in the absence or presence of compound. Next the amount of proteolysis is directly measured by fluorescence measurement at T=0 and T=120 (excitation at 320 nm and emission at 405 nm). Results are expressed in RFU (Relative Fluorescence Units), as difference between T120 and T0.

A best-fit curve is fitted by a minimum sum of squares method to the plot of %Controlmin versus compound concentration. From this an $IC_{50}$ value (inhibitory concentration causing 50% inhibition of activity) can be obtained.

LC = Median of the low control values
= Low control: Reaction without enzyme

HC = Median of the High control values
= High Control: Reaction with enzyme

$$\%Effect = 100-[(sample-LC) / (HC-LC) *100]$$

$$\%Control = (sample /HC)*100$$

$$\%Controlmin = (sample-LC) / (HC-LC) *100$$

The following exemplified compounds were tested essentially as described above and exhibited the following the activity:

**Table 5:**

| Co. Nr. | Biochemical FRET based assay $pIC_{50}$ |
|---|---|
| **1** | 7.45 |
| **2** | 7.45 |
| **3** | 7.2 |
| **4** | 6.37 |
| **5** | 6.34 |
| **6** | 7.39 |
| **7** | 7.27 |

*Cellular* αLisa *assay in SKNBE2 cells*

In two αLisa assays the levels of Abeta total and Abeta 1-42 produced and secreted into the medium of human neuroblastoma SKNBE2 cells are quantified. The assay is based on the human neuroblastoma SKNBE2 expressing the wild type Amyloid Precursor Protein (hAPP695). The compounds are diluted and added to these cells, incubated for 18 hours and then measurements of Abeta 1-42 and Abeta total are taken. Abeta total and Abeta 1-42 are measured by sandwich αLisa. αLisa is a sandwich assay using biotinylated antibody AbN/25 attached to streptavidin coated beads and antibody Ab4G8 or cAb42/26 conjugated acceptor beads for the detection of Abeta total and Abeta 1-42 respectively. In the presence of Abeta total or Abeta 1-42, the beads come into close proximity. The excitation of the donor beads provokes the release of singlet oxygen molecules that trigger a cascade of energy transfer in the acceptor beads, resulting in light emission. Light emission is measured after 1 hour incubation (excitation at 650 nm and emission at 615 nm).

A best-fit curve is fitted by a minimum sum of squares method to the plot of %Controlmin versus compound concentration. From this an $IC_{50}$ value (inhibitory concentration causing 50 % inhibition of activity) can be obtained.

LC = Median of the low control values
= Low control: cells preincubated without compound, without biotinylated Ab in the αLisa

HC = Median of the High control values
= High Control: cells preincubated without compound

$$\%Effect = 100-[(sample-LC) / (HC-LC) *100]$$

$$\%Control = (sample /HC)*100$$

$$\%Controlmin = (sample-LC) / (HC-LC) *100$$

The following exemplified compounds were tested essentially as described above and exhibited the following the activity:

**Table 6:**

| **Co. Nr.** | **Cellular αLisa assay in SKNBE2 cells Abeta 42 $pIC_{50}$** | **Cellular αLisa assay in SKNBE2 cells Abetatotal $pIC_{50}$** |
|---|---|---|
| **1** | 8.38 | 8.37 |
| **2** | 8.07 | 8.07 |
| **3** | 8.38 | 8.43 |
| **4** | 6.89 | 6.89 |
| **5** | 6.93 | 6.93 |
| **6** | 8.48 | 8.47 |
| **7** | 8.46 | 8.51 |

*Demonstration of in vivo efficacy*

Aβ peptide lowering agents of the invention can be used to treat AD in mammals such as humans or alternatively demonstrating efficacy in animal models such as, but not limited to, the mouse, rat, or guinea pig. The mammal may not be diagnosed with AD, or may not have a genetic predisposition for AD, but may be transgenic such that it overproduces and eventually deposits Aβ in a manner similar to that seen in humans afflicted with AD.

Aβ peptide lowering agents can be administered in any standard form using any standard method. For example, but not limited to, Aβ peptide lowering agents can be in the form of liquid, tablets or capsules that are taken orally or by injection. Aβ peptide lowering agents can be administered at any dose that is sufficient to significantly reduce levels of Aβ peptides in the blood, blood plasma, serum, cerebrospinal fluid (CSF), or brain.

To determine whether acute administration of an Aβ42 peptide lowering agent would reduce Aβ peptide levels in vivo, non-transgenic rodents, e.g. mice or rats were used. Animals treated with the Aβ peptide lowering agent were examined and compared to those untreated or treated with vehicle and brain levels of soluble Aβ42 and total Aβ were quantitated by standard techniques, for example, using ELISA. Treatment periods varied from hours (h) to days and were adjusted based on the results of the Aβ42 lowering once a time course of onset of effect could be established.

A typical protocol for measuring Aβ42 lowering in vivo is shown but it is only one of many variations that could be used to optimize the levels of detectable Aβ. For example, Aβ peptide lowering compounds were formulated in 20 % hydroxypropyl β cyclodextrin. The Aβ peptide lowering agents were administered as a single oral dose (p.o.) or a single subcutaneous dose (s.c.) to overnight fasted animals. After a certain time, usually 2 or 4 h (as indicated in Table 7), the animals were sacrificed and Aβ42 levels were analysed.

Blood was collected by decapitation and exsanguinations in EDTA-treated collection tubes. Blood was centrifuged at 1900 g for 10 minutes (min) at 4 °C and the plasma recovered and flash frozen for later analysis. The brain was removed from the cranium and hindbrain. The cerebellum was removed and the left and right hemisphere were separated. The left hemisphere was stored at -18 °C for quantitative analysis of test compound levels. The right hemisphere was rinsed with phosphate-buffered saline (PBS) buffer and immediately frozen on dry ice and stored at -80 °C until homogenization for biochemical assays.

Mouse brains from non-transgenic animals were resuspended in 8 volumes of 0.4 % DEA (diethylamine) /50 mM NaCl containing protease inhibitors (Roche-11873580001 or 04693159001) per gram of tissue, e.g. for 0.158 g brain, add 1.264 ml of 0.4 % DEA. All samples were homogenized in the FastPrep-24 system (MP Biomedicals) using lysing matrix D (MPBio #6913-100) at 6m/s for 20 seconds. Homogenates were centrifuged at 221.300 x g for 50 min. The resulting high speed supernatants were then transferred to fresh eppendorf tubes. Nine parts of supernatant were neutralized with 1 part 0.5 M Tris-HCl pH 6.8 and used to quantify Aßtotal and Aß42.

To quantify the amount of Aßtotal and Aß42 in the soluble fraction of the brain homogenates, Enzyme-Linked-Immunosorbent-Assays were used. Briefly, the standards (a dilution of synthetic Aβ1-40 and Aβ1-42, Bachem) were prepared in 1.5 ml Eppendorf tube in Ultraculture, with final concentrations ranging from 10000 to 0.3 pg/ml. The samples and standards were co-incubated with HRPO-labelled N-terminal antibody for Aß42 detection and with the biotinylated mid-domain antibody 4G8 for Aßtotal detection. 50 μl of conjugate/sample or conjugate/standards mixtures were then added to the antibody-coated plate (the capture antibodies selectively recognize the C-terminal end of Aß42, antibody JRF/cAß42/26, for Aß42 detection and the N-terminus of Aß, antibody JRF/rAß/2, for Aßtotal detection). The plate was allowed to incubate overnight at 4 °C in order to allow formation of the antibody-amyloid complex. Following this incubation and subsequent wash steps the ELISA for Aß42 quantification was finished by addition of Quanta Blu fluorogenic peroxidase substrate according to the manufacturer's instructions (Pierce Corp., Rockford, I1). A reading was performed after 10 to 15 min (excitation 320 nm /emission 420 nm).

For Aßtotal detection, a Streptavidine-Peroxidase-Conjugate was added, followed 60 min later by an addional wash step and addition of Quanta Blu fluorogenic peroxidase substrate according to the manufacturer's instructions (Pierce Corp., Rockford, Il). A reading was performed after 10 to 15 min (excitation 320 nm /emission 420 nm).

In this model at least 20 % Aß42 lowering compared to untreated animals would be advantageous.

The following exemplified compounds were tested essentially as described above and exhibited the following the activity:

**Table 7:**

| Co. No. | Aβ42 (%Ctrl)_Mean | Aβtotal (%Ctrl)_Mean | Dose | Route of administration | Time after administration |
|---|---|---|---|---|---|
| **1** | 62 | 59 | 10 mpk | p.o. | 2h |
| **2** | 91 | 96 | 10 mpk | p.o. | 2h |
| **7** | 53 | 55 | 30 mpk | p.o. | 4h |
| n.t. means not tested; s.c. means subcutaneous ; p.o. means oral | | | | | |

## Claims

**1.** A compound of Formula (I)

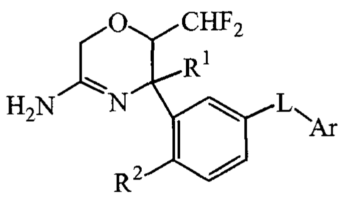

or a tautomer or a stereoisomeric form thereof, wherein

$R^1$ is $C_{1-3}$alkyl;
$R^2$ is hydrogen or fluoro;

L is a bond or -NHCO-;
Ar is selected from the group consisting of pyridinyl, pyrimidinyl and pyrazinyl, each optionally susbstituted with halo or $C_{1-3}$alkoxy;

or a pharmaceutically acceptable addition salt thereof.

2. The compound of claim 1 wherein $R^1$ is methyl or ethyl.

3. The compound of claim 2 wherein Ar is selected from 5-methoxy-pyridinyl, 5-pyrimidinyl and 5-fluoropyrazinyl.

4. The compound of claim 1 wherein $R^2$ is hydrogen or fluoro.

5. The compound of claim 1 wherein the quaternary carbon atom substituted with $R^1$ has the *R*-configuration.

6. A pharmaceutical composition comprising a therapeutically effective amount of a compound as defined in any one of claims 1 to 5 and a pharmaceutically acceptable carrier.

7. A process for preparing a pharmaceutical composition as defined in claim 6, **characterized in that** a pharmaceutically acceptable carrier is intimately mixed with a therapeutically effective amount of a compound as defined in any one of claims 1 to 5.

8. A compound as defined in any one of claims 1 to 5 for use in the treatment or prevention of Alzheimer's disease (AD), mild cognitive impairment, senility, dementia, dementia with Lewy bodies, cerebral amyloid angiopathy, multi-infarct dementia, Down's syndrome, dementia associated with stroke, dementia associated with Parkinson's disease or dementia associated with beta-amyloid.

9. A compound defined as *N*-{3-[(2R*,3R)-5-Amino-2-(difluoromethyl)-3-methyl-3,6-dihydro-2H-1,4-oxazin-3-yl]-4-fluorophenyl}-5-cyanopyridine-2-carboxamide or a pharmaceutically acceptable addition salt thereof.

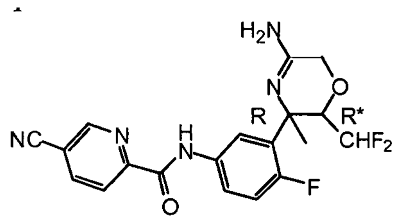

**Patentansprüche**

1. Verbindung der Formel (I)

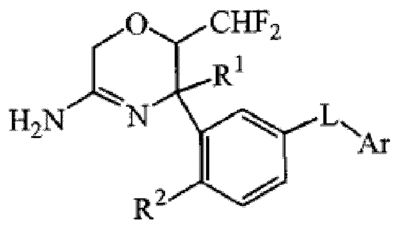

oder ein Tautomer oder eine stereoisomere Form davon, wobei

$R^1$ für $C_{1-3}$-Alkyl steht;
$R^2$ für Wasserstoff oder Fluor steht;
L für eine Bindung oder -NHCO- steht;
Ar aus der aus Pyridinyl, Pyrimidinyl und Pyrazinyl, jeweils gegebenenfalls durch Halogen oder $C_{1-3}$-Alkoxy

substituiert, bestehenden Gruppe ausgewählt ist;

oder ein pharmazeutisch unbedenkliches Additionssalz davon.

**2.** Verbindung nach Anspruch 1, wobei $R^1$ für Methyl oder Ethyl steht.

**3.** Verbindung nach Anspruch 2, wobei Ar aus 5-Methoxypyridinyl, 5-Pyrimidinyl und 5-Fluorpyrazinyl ausgewählt ist.

**4.** Verbindung nach Anspruch 1, wobei $R^2$ für Wasserstoff oder Fluor steht.

**5.** Verbindung nach Anspruch 1, wobei das durch $R^1$ substituierte quartäre Kohlenstoffatom die *R*-Konfiguration hat.

**6.** Pharmazeutische Zusammensetzung, umfassend eine therapeutisch wirksame Menge einer wie in einem der Ansprüche 1 bis 5 definierten Verbindung und einen pharmazeutisch unbedenklichen Träger.

**7.** Verfahren zur Herstellung einer wie in Anspruch 6 definierten pharmazeutischen Zusammensetzung, **dadurch gekennzeichnet, dass** man einen pharmazeutisch unbedenklichen Träger innig mit einer therapeutisch wirksamen Menge einer wie in einem der Ansprüche 1 bis 5 definierten Verbindung mischt.

**8.** Verbindung, definiert wie in einem der Ansprüche 1 bis 5, zur Verwendung bei der Behandlung oder Prävention von Alzheimer-Krankheit (AD), milder kognitiver Störung, Senilität, Demenz, Demenz mit Lewy-Körperchen, zerebraler Amyloidangiopathie, Multiinfarktdemenz, Down-Syndrom, mit Schlaganfall assoziierter Demenz, mit Parkinson-Krankheit assoziierter Demenz oder mit beta-Amyloid assoziierter Demenz.

**9.** Verbindung, definiert als *N*-{3-[(2R*,3R)-5-Amino-2-(difluormethyl)-3-methyl-3,6-dihydro-2H-1,4-oxazin-3-yl]-4-fluorphenyl}-5-cyanopyridin-2-carboxamid, oder ein pharmazeutisch unbedenkliches Additionssalz davon.

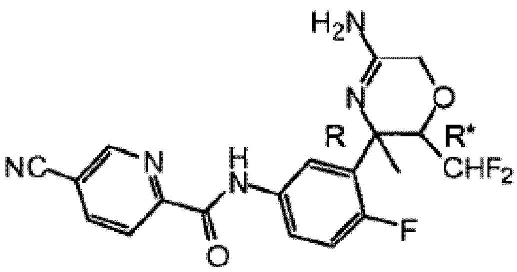

## Revendications

**1.** Composé de Formule (I)

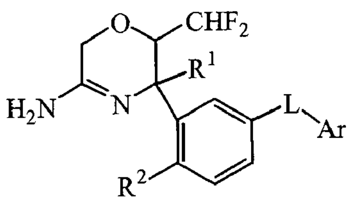

ou un tautomère ou une forme stéréoisomère de celui-ci, dans laquelle

$R^1$ est $C_{1-3}$alkyle ;
$R^2$ est hydrogène ou fluoro ;
L est une liaison ou -NHCO- ;
Ar est choisi dans le groupe constitué par pyridinyle, pyrimidinyle et pyrazinyle, chacun éventuellement substitué par halogéno ou $C_{1-3}$alcoxy ;

ou un sel d'addition pharmaceutiquement acceptable de celui-ci.

**2.** Composé selon la revendication 1, dans lequel $R^1$ est méthyle ou éthyle.

**3.** Composé selon la revendication 2, dans lequel Ar est choisi parmi 5-méthoxypyridinyle, 5-pyrimidinyle et 5-fluoropyrazinyle.

**4.** Composé selon la revendication 1, dans lequel $R^2$ est hydrogène ou fluoro.

**5.** Composé selon la revendication 1, dans lequel l'atome de carbone quaternaire substitué par $R^1$ présente une configuration en R.

**6.** Composition pharmaceutique comprenant une quantité thérapeutiquement efficace d'un composé tel que défini selon l'une quelconque des revendications 1 à 5 et un véhicule pharmaceutiquement acceptable.

**7.** Procédé de préparation d'une composition pharmaceutique telle que définie selon la revendication 6, **caractérisé en ce qu'**un véhicule pharmaceutiquement acceptable est mélangé intimement avec une quantité thérapeutiquement efficace d'un composé tel que défini selon l'une quelconque des revendications 1 à 5.

**8.** Composé tel que défini selon l'une quelconque des revendications 1 à 5, pour une utilisation dans le traitement ou la prévention de la maladie d'Alzheimer (AD), le trouble cognitif léger, la sénilité, la démence, la démence à corps de Lewy, l'angiopathie amyloïde cérébrale, la démence par infarctus multiples, la trisomie 21, la démence associée aux accidents vasculaires cérébraux, la démence associée à la maladie de Parkinson ou la démence associée aux bêta-amyloïdes.

**9.** Composé défini comme le N-{3-[(2R*,3R)-5-amino-2-(difluorométhyl)-3-méthyl-3,6-dihydro-2H-1,4-oxazin-3-yl]-4-fluorophényl}-5-cyanopyridine-2-carboxamide ou un sel d'addition pharmaceutiquement acceptable de celui-ci.

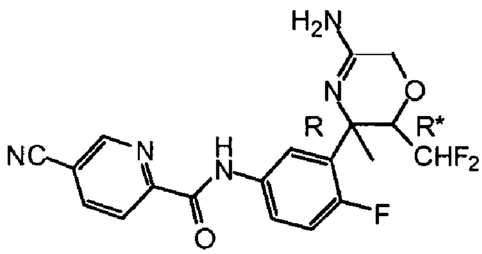

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2011009943 A
- WO 2011020806 A